# FASCICULE DE BREVET EUROPEEN

(11) **EP 1 370 231 B1**
(45) Date de publication et mention de la délivrance du brevet: **08.08.2007**
(21) Numéro de dépôt: 01271203.0
(22) Date de dépôt: 20.11.2001
(51) Int. Cl.: A61Q 17/04, A61K 8/37, A61K 8/40, A61K 8/35

(54) **COMPOSITION FILTRANTE CONTENANT UN FILTRE DU TYPE DERIVE DU DIBENZOYLMETHANE ET UN FILTRE DU TYPE 4,4-DIARYLBUTADIENE**
FILTERZUSAMMENSETZUNG MIT EINEM FILTER DES VON DIBENZOYLMETHAN ABSTAMMENDEN TYPS UND EINEM 4,4-DIARYLBUTADIEN-FILTER
FILTERING COMPOSITION CONTAINING A FILTER OF THE TYPE DERIVED FROM DIBENZOYLMETHANE AND A 4,4-DIARYLBUTADIENE TYPE FILTER

(30) Priorité: 18.12.2000 FR 0016518
(43) Date de publication de la demande: 17.12.2003
(73) Titulaire: L'ORÉAL, 75008 Paris (FR)
(72) Inventeur: CANDAU, Didier, F-91570 Bièvres (FR)
(74) Mandataire: Miszputen, Laurent
(86) Numéro de dépôt international: PCT/FR2001/003635
(87) Numéro de publication internationale: WO 2002/049595

(56) Documents cités:
- EP-A- 0 114 607
- EP-A- 0 967 200
- EP-A- 1 008 586
- EP-A- 1 133 981
- DE-A- 10 007 017
- DE-A- 19 746 654
- DE-A- 19 755 649
- FR-A- 2 326 405
- FR-A- 2 440 933
- FR-A- 2 658 075

## Description

L'invention se rapporte à une composition cosmétique ou dermatologique, à usage topique, destinée à la photoprotection de la peau et des cheveux comprenant en association un filtre UV du type dérivé de dibenzoylméthane et un composé 4,4-diarylbutadiène.

L'invention se rapporte également à un procédé pour améliorer la stabilité d'au moins un dérivé du dibenzoylméthane vis-à-vis du rayonnement UV consistant à associer au dit dérivé de dibenzoylméthane une quantité efficace d'au moins un composé 4,4-diarylbutadiène.

On sait que les radiations lumineuses de longueurs d'onde comprises entre 280 nm et 400 nm permettent le brunissement de l'épiderme humain, et que les rayons de longueurs d'onde plus particulièrement comprises entre 280 et 320 nm, connus sous la dénomination UV-B, provoquent des érythèmes et des brûlures cutanées qui peuvent nuire au développement du bronzage naturel. Pour ces raisons ainsi que pour des raisons esthétiques, il existe une demande constante de moyens de contrôle de ce bronzage naturel en vue de contrôler ainsi la couleur de la peau ; il convient donc de filtrer ce rayonnement UV-B.

On sait également que les rayons UV-A, de longueurs d'onde comprises entre 320 et 400 nm, qui provoquent le brunissement de la peau, sont susceptibles d'induire une altération de celle-ci, notamment dans le cas d'une peau sensible ou d'une peau continuellement exposée au rayonnement solaire. Les rayons UV-A provoquent en particulier une perte d'élasticité de la peau et l'apparition de rides conduisant à un vieillissement cutané prématuré. Ils favorisent le déclenchement de la réaction érythémateuse ou amplifient cette réaction chez certains sujets et peuvent même être à l'origine de réactions phototoxiques ou photo-allergiques. Ainsi, pour des raisons esthétiques et cosmétiques telles que la conservation de l'élasticité naturelle de la peau par exemple, de plus en plus de gens désirent contrôler l'effet des rayons UV-A sur leur peau. Il est donc souhaitable de filtrer aussi le rayonnement UV-A.

A cet égard, une famille de filtres UV-A particulièrement intéressante est actuellement constituée par les dérivés du dibenzoylméthane, et notamment le 4-ter-butyl-4'-méthoxydibenzoyl méthane, qui présentent en effet un fort pouvoir d'absorption intrinsèque. Ces dérivés du dibenzoylméthane, qui sont maintenant des produits bien connus en soi à titre de filtres actifs dans les UV-A, sont notamment décrits dans les demandes de brevets français FR-A-2326405 et FR-A-2440933, ainsi que dans la demande de brevet européen EP-A-0114607 ; le 4-ter-butyl- 4'-méthoxydibenzoyl méthane est par ailleurs actuellement proposé à la vente sous la dénomination commerciale de "PARSOL 1789" par la Société HOFFMANN LAROCHE.

Malheureusement, il se trouve que les dérivés du dibenzoylméthane sont des produits relativement sensibles au rayonnement ultraviolet (surtout UV-A), c'est-à-dire, plus précisément, qu'ils présentent une fâcheuse tendance à se dégrader plus ou moins rapidement sous l'action de ce dernier. Ainsi, ce manque substantiel de stabilité photochimique des dérivés du dibenzoylméthane face au rayonnement ultraviolet auquel ils sont par nature destinés à être soumis, ne permet pas de garantir une protection constante durant une exposition solaire prolongée, de sorte que des applications répétées à intervalles de temps réguliers et rapprochés doivent être effectuées par l'utilisateur pour obtenir une protection efficace de la peau contre les rayons UV.

La photostabilisation des dérivés du dibenzoylméthane vis-à-vis du rayonnement UV constitue, à ce jour, un problème qui n'a pas encore été résolu de manière complètement satisfaisante.

On connaît dans les demandes de brevet EP0967200, DE19746654, DE19755649, EP1008 586, DE 100 07 017, EP 1133980 et EP 1133981 des compositions solaires à base de 4,4-diarylbutadiènes pouvant contenir d'autres filtres complémentaires comme les dérivés de dibenzoylméthane.

Or, la Demanderesse vient maintenant de découvrir, de façon surprenante, qu'en associant aux dérivés du dibenzoylméthane mentionnés ci-dessus une quantité d'au moins un 4,4-diarylbutadiène, il était possible d'améliorer de manière substantielle et remarquable, la stabilité photochimique (ou photostabilité) de ces mêmes dérivés du dibenzoylméthane.

Ainsi, conformément à l'un des objets de la présente invention, il est maintenant proposé une nouvelle composition cosmétique ou dermatologique, à usage topique, caractérisée par le fait qu'elle comprend au moins, dans un support cosmétiquement acceptable :
(a) au moins un filtre UV du type dérivé du dibenzoylméthane et
(b) au moins un composé 4,4-diarylbutadiène ; le rapport en poids du composé 4,4-diarylbutadiène sur le dérivé de dibenzoylméthane étant supérieur à 2,5 et ladite composition ne contenant pas de dérivé de cinnamate.

La présente invention a également pour objet un procédé pour améliorer la stabilité d'au moins un dérivé du dibenzoylméthane vis-à-vis du rayonnement UV consistant à associer au dit dérivé de dibenzoylméthane une quantité efficace d'au moins un composé 4,4-diarylbutadiène.

Par quantité efficace de 4,4-diarylbutadiène conforme à l'invention, on entend une quantité suffisante pour obtenir une amélioration notable et significative de la photostabilité du ou des dérivés du dibenzoylméthane de la composition cosmétique photoprotectrice. Cette quantité minimale en agent photostabilisant à mettre en oeuvre, qui peut varier selon la nature du support cosmétiquement acceptable retenu pour la composition, peut être déterminée sans aucune difficulté au moyen d'un test classique de mesure de photostabilité.

Par composé 4,4-diarylbutadiène conforme à l'invention, on entend toute molécule comportant au moins un groupe chromophore 4,4-diarylbutadiène.

Celle-ci peut se présenter sous forme de composé simple, d'oligomère ou de polymère possédant sur la chaîne des greffons contenant le groupe chromophore.

La présente invention a également enfin pour objet l'utilisation d'un composé 4,4-diarylbutadiène dans la préparation d'une composition cosmétique ou dermatologique comprenant au moins un dérivé du dibenzoylméthane dans le but de d'améliorer la stabilité vis-à-vis des rayons UV dudit dérivé de dibenzoylméthane contenu.

D'autres caractéristiques, aspects et avantages de la présente invention apparaîtront à la lecture de la description détaillée qui va suivre.

Parmi les composés 4,4-diarylbutadiènes conformes à l'invention préférés, on peut choisir les composés répondant à la formule (I) suivante : 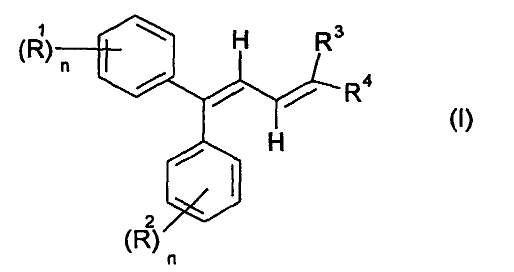 dans laquelle le système diène est de configuration Z,Z ; Z,E ; E,Z ou E,E ou des mélanges desdites configurations et où:
- R¹ et R², identiques ou différents, désignent hydrogène, un radical alkyle en C₁-C₂₀, linéaire ou ramifié ; un radical alcényle en C₂- C₁₀ ; un radical alcoxy en C₁-C₁₂ ; un radical cycloalkyle en C₃-C₁₀ ; un radical cycloalcényle en C₃-C₁₀; un radical alcoxycarbonyle en C₁-C₂₀ linéaire ou ramifié ; un radical monoalkylamino en C₁-C₁₂, linéaire ou ramifié ; un radical dialkylamino en C₁-C₁₂, linéaire ou ramifié ; un aryle ; un hétéroaryle ou un substituant hydrosolubilisant choisi parmi un groupe carboxylate, un groupe sulfonate ou un reste ammonium ;
- R désigne un groupe COOR⁵ ; COR⁵ ; CONR⁵R⁶ ; CN ; un radical alkyle en C₁-C₂₀, linéaire ou ramifié ; un radical alcényle en C₂- C₁₀ ; un radical cycloalkyle en C₃-C₁₀; un radical bicycloalkyle en C₇-C₁₀ ; un radical cycloalcényle en C₃-C₁₀ ; un radical bicycloalcényle en C₇-C₁₀ ; un aryle en C₆-C₁₈ ; un hétéroaryle en C₃-C₇ ;
- R⁴ désigne un groupe COOR⁶ ; COR⁶; CONR⁵R⁶ ; CN ; un radical alkyle en C₁-C₂₀, linéaire ou ramifié ; un radical alcényle en C₂- C₁₀ ; un radical cycloalkyle en C₃-C₁₀ ; un radical bicycloalkyle en C₇-C₁₀ ; un radical cycloalcényle en C₃-C₁₀ ; un radical bicycloalcényle en C₇-C₁₀ ; un aryle ; un hétéroaryle ;
- R⁵ et R⁶, identiques ou différents, désignent hydrogène ; [X]ₚ-R⁷, C₁-C₆-alkylène-SO₃Y; C₁-C₆-alkylène-PO₃Y; C₁-C₆-alkylène-N(R⁸)₃⁺ A⁻; un radical alkyle en C₁-C₂₀, linéaire ou ramifié ; un radical alcényle en C₂₋C₁₀; un radical cycloalkyle en C₃₋C₁₀; un radical bicycloalkyle en C₇-C₁₀; un radical bicycloalcényle en C₃-C₁₀; un radical cycloalcényle en C₇₋C₁₀; un aryle ; un hétéroaryle ;
- X désigne un groupe -CH₂-CH₂-Z-, -CH₂CH₂CH₂Z-, -CH(CH₃)-CH₂-Z-, -CH₂-CH₂-CH₂-CH₂-Z-, -CH₂-CH(CH₂CH₃)-Z- ;
- A désigne Cl, Br, I, SO₄R⁹ ;
- Y désigne hydrogène, Na⁺, K⁺, Mg²⁺, Ca²⁺, Li⁺, Al³⁺, -N(R⁸)₄⁺
- Z désigne O ou NH ;
- R⁷ et R⁸ identiques ou différents, désignent hydrogène, un radical alkyle en C₁-C₆, linéaire ou ramifié ; un radical alcényle en C₂- C₆, linéaire ou ramifié ; un radical acyle en C₁-C₆ linéaire ou ramifié ;
- R⁹ désigne hydrogène, un radical alkyle en C₁-C₆, linéaire ou ramifié ; un radical alcényle en C₂- C₆ ;
- n varie de 1 à 3 ;
- p varie de 0 à 150.

Comme radicaux alkyle en C₁-C₂₀, on peut citer par exemple : méthyle, éthyle, n-propyle, 1-méthyléthyle, n-butyle, 1-méthylpropyle, 2-méthylpropyle, 1,1-diméthyléthyle, n-pentyle, 1-méthylbutyle, 2-méthylbutyle, 3-méthylbutyle, 2,2-diméthylpropyle, 1-éthylpropyle, n-hexyle, 1,1-diméthylpropyle, 1,2-diméthylpropyle, 1-méthylpentyle, 2-méthylpentyle, 3-méthylpentyle, 4-méthylpentyle, 1,1-diméthylbutyle, 1,2-diméthylbutyle, 1,3-diméthylbutyle, 2,2-diméthylbutyle, 2,3-diméthylbutyle, 3,3-diméthylbutyle, 1-éthylbutyle, 2-éthylbutyle, 1,2,2-triméthylpropyle, 1-éthyl-1-méthylpropyle, 1-éthyl-2-méthylpropyle, n-heptyle, n-octyle, n-nonyle, n-décyle, n-undécyle, n-dodécyle, n-tridécyle, n-tétradécyle, n-pentadécyle, n-hexadécyle, n-heptadécyle, n-octadécyle, n-nonadécyle ou n-eicosyle.

Comme groupes alcènyle en C₂-C₁₀, on peut citer par exemple : éthènyle, n-propènyle, 1-méthyléthènyle, n-butènyle, 1-méthylpropènyle, 2-méthylpropènyle, 1,1-diméthyléthènyle, n-pentènyle, 1-méthylbutényle, 2-méthylbutènyle, 3-méthylbutènyle, 2,2-diméthylpropènyle, 1-éthylpropènyle, n-hexènyle, 1,1-diméthylpropènyle, 1,2-diméthylpropènyle, 1-méthylpentènyle, 2-méthylpentènyle, 3-méthylpentènyle, 4-méthylpentènyle,1,1-diméthylbutènyle,1,2-diméthylbutènyle,1,3-diméthylbutènyl e,2,2-diméthylbutènyle, 2,3-diméthylbutènyle, 3,3-diméthylbutènyle, 1-éthylbutènyle, 2-éthylbutènyle, 1, 1,2-triméthytpropènyle, 1,2,2-triméthylpropènyle, 1-éthyl-1-méthylpropènyle, 1-éthyl-2-méthylpropènyle, n-heptènyle, n-octènyle, n-nonènyle, n-décènyle.

Comme radicaux alcoxy en C₁-C₁₂, on peut citer : méthoxy, n-propoxy, 1-méthylpropoxy, 1-méthyléthoxy, n-pentoxy, 3-méthylbutoxy, 2,2-diméthylpropoxy, 1 -méthyl- 1-éthylpropoxy, octoxy, éthoxy, n-propoxy, n-butoxy, 2-méthylpropoxy, 1,1-diméthylpropoxy, hexoxy, heptoxy, 2-éthylhexoxy.

Comme radicaux cycloalkyles en C₃-C₁₀, on peut citer par exemple : cyclopropyle, cyclobutyle, cyclopentyle, cyclohexyle, cycloheptyle, 1-méthylcyclopropyle, 1-éthylcyclopropyle, 1-propylcyclopropyle, 1-butylcyclopropyle, 1-pentylcyclopropyle, 1-méthyl-1-butylcyclopropyle, 1,2-diméthylcyclypropyle, 1-méthyl-2-éthylcyclopropyle, cyclooctyle, cyclononyle ou cyclodécyle.

Comme radicaux cycloalcènyles en C₃-C₁₀ ayant une ou plusieurs doubles liaisons, on peut citer : cyclobutènyle, cyclopentènyle, cyclopentadiènyle, cyclohexènyle, 1,3-cyclohexadiènyle, 1,4-cyclohexadiènyle, cycloheptènyle, cycloheptatriènyle, cyclooctènyle, 1,5-cyclooctadiènyle, cyclooctétraènyle, cyclononènyle ou cyclodécènyle.

Les radicaux cycloalkyles ou cycloalcényles peuvent comporter un ou plusieurs substituants (de préférence de 1 à 3) chois par exemple parmi halogène comme chlore, fluor ou brome ; cyano ; nitro; amino ; C₁-C₄-alkylamino ; C₁-C₄ dialkylamino ; C₁-C₄alkyle ; C₁-C₄-alcoxy ; hydroxy ; ils peuvent également comporter de 1 à 3 hétéroatomes comme souffre, oxygène ou azote dont les valences libres peuvent être saturées par un hydrogène ou un radical alkyle en C₁-C₄.

Comme radicaux acyle, on peut citer par exemple formyle, acétyle, propionyle, ou n-butyryle.

Les groupes bicycloalkyles ou bicycloalcényles sont choisis par exemple parmi les terpènes bicycliques comme les dérivés de pinane, de bornane, de pinène ou de camphre ou d'adamantane.

Les groupes aryles sont de préférence choisis parmi les cycles phényle ou naphtyle, lesquels pouvant comporter un ou plusieurs substituants (de préférence de 1 à 3) chois par exemple parmi halogène comme chlore, fluor ou brome ; cyano; nitro ; amino ; C₁-C₄-alkylamino ; C₁-C₄ dialkylamino ; C₁-C₄alkyle ; C₁-C₄-alcoxy ; hydroxy. On préfère plus particulièrement phényle, méthoxyphényle et naphtyle.

Les groupes hétéroaryles comportent en général un ou plusieurs hétéroatomes choisis parmi souffre, oxygène ou azote.

Les groupes hydrosolubilisants sont par exemple des groupes carboxylates, sulfonates et plus particulièrement leurs sels avec des cations physiologiquement acceptables comme les sels de métaux alcalins ou les sels de trialkylammonium comme les sels de tri(hydroxyalkyl)ammonium ou de 2-méthylpropan-1-ol-2-ammonium. On peut également citer les groupes ammonium comme les alkylammoniums et leurs formes salifiées avec des anions physiologiquement acceptables.

Les composés de formule (I) préférentiels sont choisis parmi ceux de formule (la) suivante : 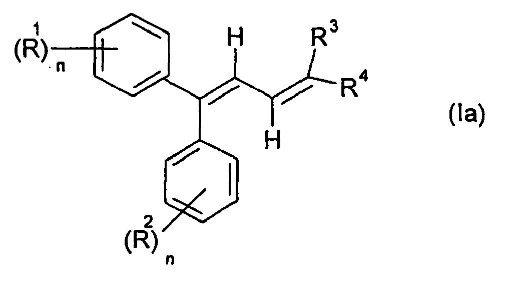 dans laquelle le système diène est de configuration Z,Z ; Z,E ; E,Z ou E,E ou des mélanges desdites configurations et où :
- R¹ et R² identiques ou différents, désignent hydrogène, un radical alkyle en C₁-C₈, un radical alcoxy en C₁-C₈; un substituant hydrosolubilisant choisi parmi un groupe carboxylate, un groupe sulfonate ou un reste ammonium ;
- R³ désigne un groupe COOR⁵; CONR⁵R⁶; CN ;
- R⁴ désigne un groupe COOR⁶ ; CONR⁵R⁶ ;
- R⁵ désigne hydrogène ; [X]ₚ-R⁷; C₁-C₆-alkylène-SO₃Y; C₁-C₆-alkylène-N(R⁸)h⁺ A⁻;
- R⁶ désigne [X]p-R⁷ ; C₁-C₆-alkylène-SO₃Y ; C₁-C₆-alkylène-N(R⁸)3⁺ A⁻ ;
- X désigne un groupe -CH₂-CH₂-O-, -CH₂CH₂CH₂O-, -CH(CH₃)-CH₂-O-,
- A désigne Cl, Br, l, SO₄R^{9;}
- Y désigne hydrogène, Na⁺, K⁺, Mg²⁺, Ca²⁺, Li⁺, Al³⁺, -N(R⁸)₄⁺
- R⁷ , R et R⁹ identiques ou différents, désignent hydrogène, un radical alkyle en C₁-C₃, linéaire ou ramifié ;
- n varie de 1 à 3
- p varie de 0 à 50;

Les composés de formule (1) encore plus préférentiels sont choisis parmi ceux répondant à la formule (1b) suivante : 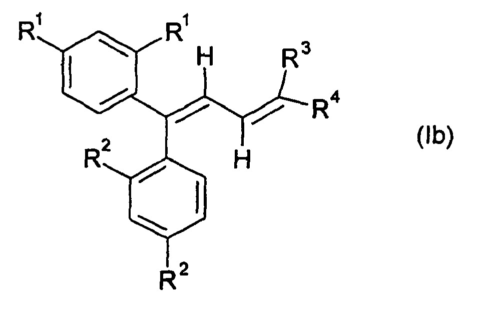 dans laquelle le système diène est de configuration Z,Z ; Z,E ; E,Z ou E,E ou des mélanges desdites configurations et où :
- R¹ et R², identiques ou différents, désignent hydrogène, un radical alkyle en C₁-C₈; un radical alcoxy en C₁-C₈ ;
- R³ désigne un groupe COOR⁵ ; CONR⁵R⁶; CN ;
- R⁴ désigne un groupe COOR⁶ ; CONR⁵R⁶ ;
- R⁵ désigne hydrogène ; [X]ₚ-R⁷ ; C₁-C₆-alkylène-SO₃Y ; C₁-C₆-alkylène-N(R⁸)₃⁺ A' ;
- R⁶ désigne [X]p-R⁷ ; C₁-C₆-alkyléne-SO₃Y ; C₁-C₆-alkylène-N(R⁸)₃⁺ A⁻ ;
- X désigne un groupe -CH₂-CH₂-O-, -CH₂CH₂CH₂O-, -CH(CH₃)-CH₂-O-,
- A désigne Cl, Br, I, SO₄R⁹ ; Mg²⁺, Ca²⁺ Al³⁺,
- Y désigne hydrogène, Na⁺, K⁺, Mg²⁺, Ca²⁺, Li⁺, Al³⁺, -N(R⁸)₄⁺
- R⁷ , R et R⁹ identiques ou différents, désignent hydrogène, un radical alkyle en C₁-C₃, linéaire ou ramifié ;
- p varie de 0 à 50.

Les composés de formule (I) encore plus préférentiels sont choisis parmi ceux répondant à la formule (Ic) suivante : 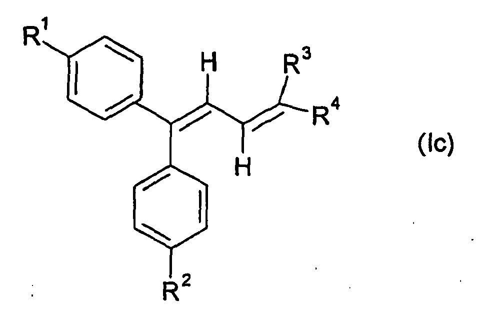 dans laquelle le système diène est de configuration Z,Z ; Z,E ; E,Z ou E,E ou des mélanges desdites configurations et où :
- R¹ et R², identiques ou différents, désignent hydrogène, un radical alkyle en C₁-C₈ un radical alcoxy en C₁-Ca ;
- R³ désigne un groupe COOR⁵; CONR⁵R⁶ ; CN ;
- R⁴ désigne un groupe COOR⁶ ; CONR⁵R⁶ ;
- R⁵ désigne hydrogène ; [X]p-R⁷; C₁-C₆-alkylène-SO₃Y; C₁-C₆-alkylène-N(R⁶)₃⁺ A⁻;
- R⁶ désigne [X]p-R⁷; C₁-C₆-alkylène-SO₃Y ; C₁-C₆-alkylène-N(R⁸)₃⁺ A⁻;
- X désigne un groupe -CH₂-CH₂-O-, -CH₂CH₂CH₂O-, -CH(CH₃)-CH₂-O-,
- A désigne Cl, Br, I, SO₄R⁹;
- Y désigne hydrogène, Na⁺, K⁺, Mg²⁺, Ca²⁺, Li⁺, Al³⁺, -N(R⁸)₄⁺
- R⁷, R et R⁹ identiques ou différents, désignent hydrogène, un radical alkyle en C₁-C₃, linéaire ou ramifié ;
- p varie de 0 à 50.

Les composés de formule (I) encore plus particulièrement préférés sont choisis parmi les composés suivants :

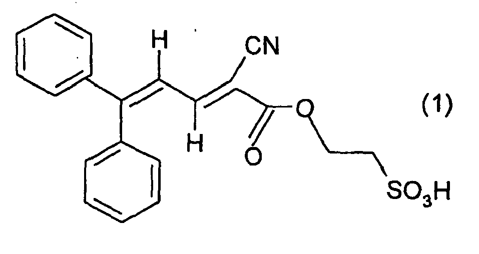

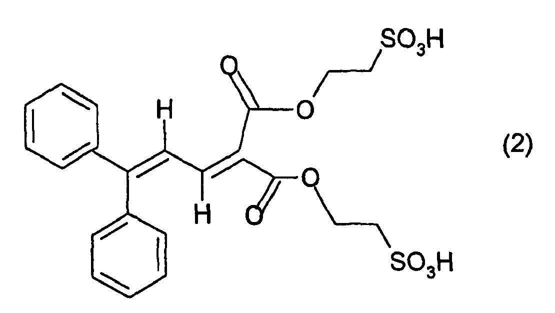

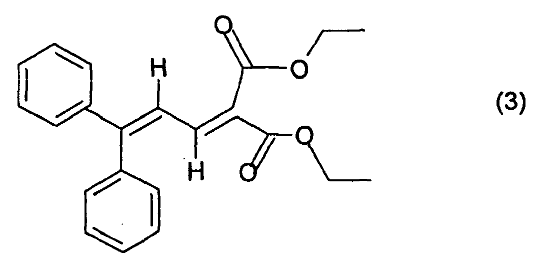

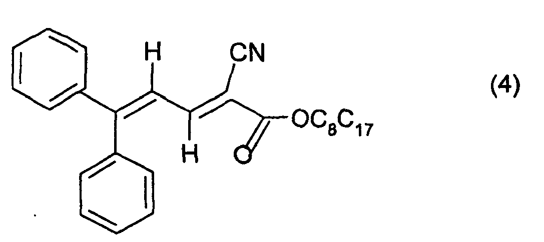

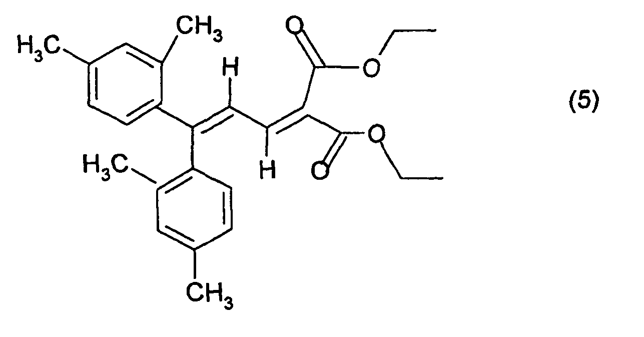

Les composés de formule (I) tels que définis ci-dessus sont connus en eux-mêmes et leurs structures et leurs synthèses sont décrites dans les demandes de brevet EP0967200, DE 19746654 et DE19755649, (faisant partie intégrante du contenu de la description).

Parmi les composés 4,4-diarylbutadiènes conformes à l'invention préférés, on peut également citer les oligomères répondant à la formule (II) suivante : 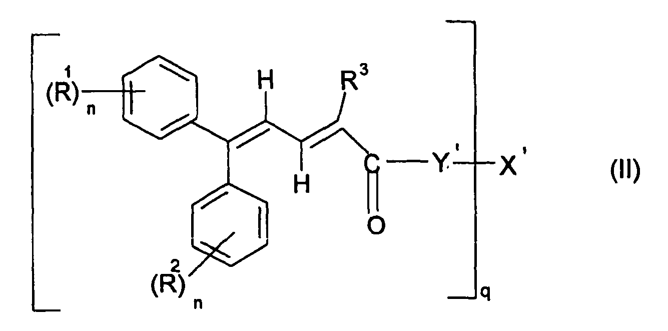 dans laquelle le système diène est de configuration Z,Z ; Z,E ; E,Z ou E,E ou des mélanges desdites configurations et où :
- R¹, R² R³ et n ont les mêmes significations indiquées dans la formule (I) précédente ;
- Y' désigne un groupe -O- ou -NR¹⁰-
- R¹⁰ désigne hydrogène ; un radical alkyle en C₁-C₂₀, linéaire ou ramifié ; un radical alcényle en C₂- C₁₀; un radical cycloalkyle en C₃-C₁₀; un radical bicycloalkyle en C₇-C₁₀; un radical cycloalcényle en C₃-C₁₀; ; un radical bicycloalcényle en C₇-C₁₀ ; un aryle ; un hétéroaryle ;
- X' désigne un reste de polyol linéaire ou ramifié, aliphatique ou cycloaliphatique comprenant de 2 à10 groupes hydroxy et de valence q ; la chaîne carbonée dudit reste pouvant être interrompue par un ou plusieurs atomes de souffre ou d'oxygène ; un ou plusieurs groupes imines ; un ou plusieurs alkylimino en C₁-C₄ ;
- q varie de 2 à 10.

X' est un reste polyol contenant de 2 à 10 groupes hydroxyles et notamment :

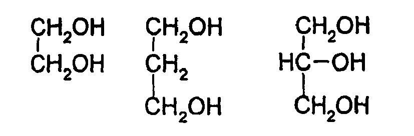

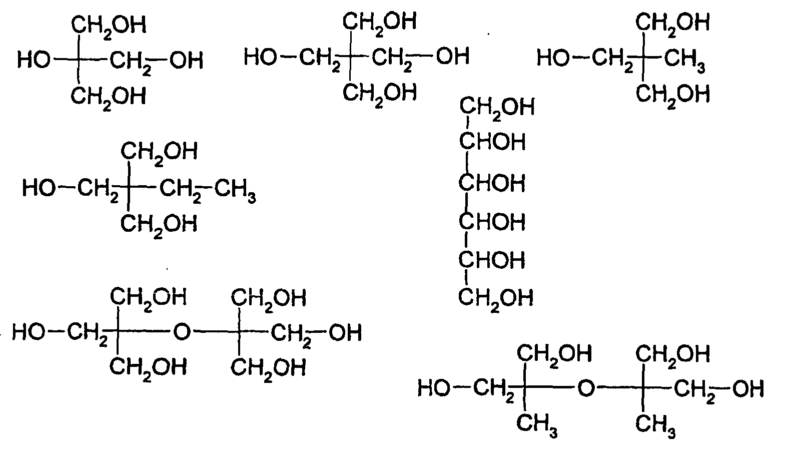

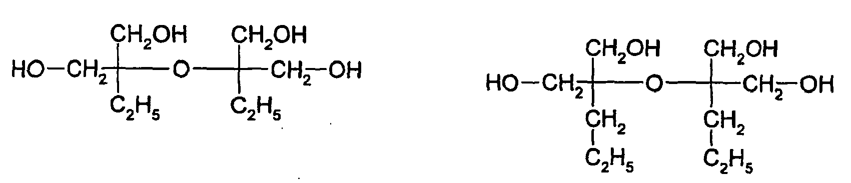

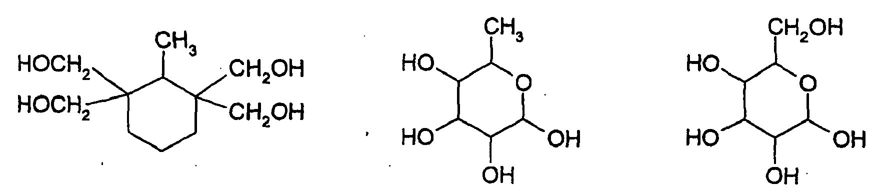

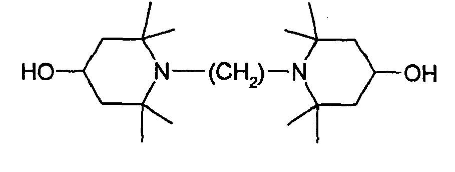

Les composés plus préférentiels de formule (II) sont ceux pour lesquels :
- R¹ et R², identiques ou différents, désignent hydrogène, un radical alkyle en C₁-C₁₂; un radical alcoxy en C₁-C₈ ; un substituant hydrosolubilisant choisi parmi un groupe carboxylate, un groupe sulfonate ou un reste ammonium ;
- R désigne un groupe COOR⁵ ; CONR⁵R⁶ ; CN ; un radical cycloalkyle en C₃-C₁₀ ; un radical bicycloalkyle en C₇-C₁₀ ;
- R⁵ et R⁶ , identiques ou différents, désignent un radical alkyle en C₁-C₂₀, linéaire ou ramifié ; un radical cycloalkyle en C₃-C₁₀ ; un radical bicycloalkyle en C₇-C₁₀ ; naphtyle ou phényle éventuellement substitué ;
- X' désigne un reste de polyol comprenant de 2 à 6 groupes hydroxy et plus particulièrement de 2 à 4.

Les composés encore plus préférentiels de formule (II) sont ceux pour lesquels :
- X' désigne un reste d'éthanol ou de pentaerythrol.

Les composés de formule (II) encore plus particulièrement préférés sont choisis parmi les composés suivants :

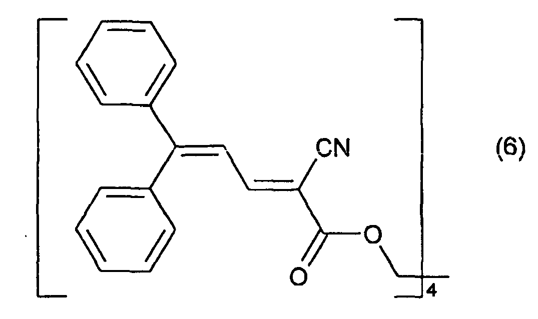

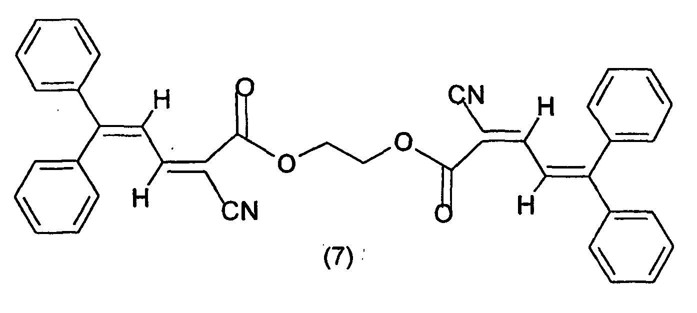

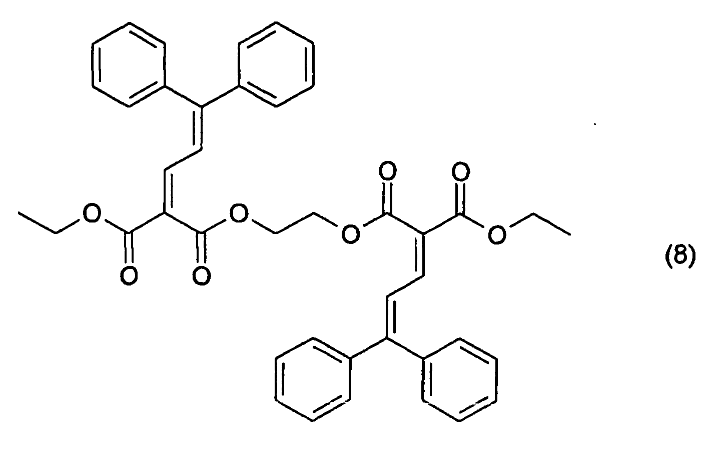

Les composés de formule (II) tels que définis ci-dessus sont connus en eux-mêmes et leurs structures et leurs synthèses sont décrites dans la demande de brevet EP-A-1008586 (faisant partie intégrante du contenu de la description).

Les composés 4,4-diarylbutadiène conformes à l'invention sont présents de préférence dans la composition de l'invention dans des proportions allant de 0,5 % à 15% en poids, plus préférentiellement de 1 à 10% en poids par rapport au poids total de la composition.

Comme indiqué précédemment, les dérivés du dibenzoylméthane destinés à être photostabilisés dans le cadre de la présente invention sont des produits déjà bien connus en soi et décrits notamment dans les documents FR2326405, FR2440933 et EP0114607 précités, documents dont les enseignements sont, pour ce qui touche à la définition même de ces produits, totalement inclus à titre de références dans la présente description.

Selon la présente invention, on peut bien entendu mettre en oeuvre un ou plusieurs dérivés du dibenzoylméthane.

Parmi les dérivés du dibenzoylméthane rentrant particulièrement bien dans le cadre de la présente invention, on peut notamment citer, de manière non limitative :
- le 2-méthyldibenzoylméthane
- le 4-méthyldibenzoylméthane
- le 4-isopropyldibenzoylméthane
- le 4-tert.-butyldibenzoylméthane
- le 2,4-diméthyldibenzoylméthane
- le 2,5-diméthyldibenzoylméthane
- le 4,4'-diisopropyldibenzoylméthane
- le 4,4'-diméthoxydibenzoylméthane
- le 4-tert.-butyl-4'-méthoxydibenzoylméthane
- le 2-méthyl-5-isopropyl-4'-méthoxydibenzoylméthane
- le 2-méthyl-5-tert.-butyl-4'-méthoxydibenzoylméthane
- le 2,4-diméthyl-4'-méthoxydibenzoylméthane
- le 2,6-diméthyl-4-tert.-butyl-4'-méthoxydibenzoylméthane

Parmi les dérivés du dibenzoylméthane mentionnés ci-dessus, on préfère tout particulièrement, selon la présente invention, mettre en oeuvre le 4-(ter.-butyl) 4'-méthoxy dibenzoylméthane, notamment celui proposé à la vente sous la dénomination commerciale de "PARSOL 1789" par la Société HOFFMANN LAROCHE, ce filtre répondant donc à la formule développée suivante :

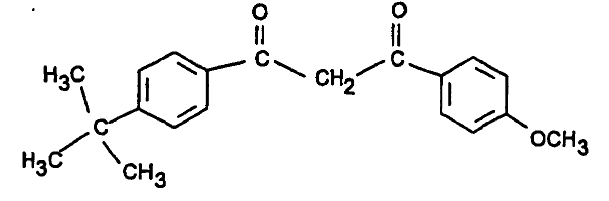

Un autre dérivé du dibenzoylméthane préféré selon la présente invention est le 4-isopropyl-dibenzoylméthane, filtre vendu sous la dénomination de "EUSOLEX 8020" par la Société MERCK, et répondant à la formule développée suivante :

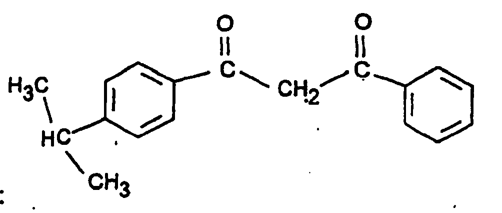

Le ou les dérivés du dibenzoylméthane peuvent être présents dans les compositions conformes à l'invention à des teneurs qui varient de préférence de de 0,5 à 15% en poids et plus particulièrement de 1 % à 10 % en poids, par rapport au poids total de la composition.

Les compositions conformes à l'invention peuvent comporter en plus d'autres filtres UV organiques complémentaires actifs dans l'UVA et/ou l'UVB (absorbeurs), hydrosolubles ou liposolubles ou bien insolubles dans les solvants cosmétiques couramment utilisés.

Les filtres UV organiques complémentaires sont notamment choisis parmi les anthranilates; les dérivés salicyliques, les dérivés du camphre ; les dérivés de triàzine tels que ceux décrits dans les demandes de brevet US 4367390, EP863145, EP517104, EP570838, EP796851, -EP775698, EP878469 et EP933376 ; les dérivés de la benzophénone ; les dérivés de (β,β'-diphénylacrylate, les dérivés de benzotriazole, les dérivés de benzimidazole ; les imidazolines; les dérivés de l'acide p-aminobenzoïque (PABA) ; les dérivés de méthylène bis-(hydroxyphényl benzotriazole) tels que décrits dans les demandes US5,237,071, US5,166,355, GB2303549, DE19726184 et EP893119 ; les polymères filtres et silicones filtres tels que ceux décrits notamment dans la demande WO93/04665; les dimères dérivés d'α-alkylstyrène tels que ceux décrits **dans la demande de brevet** DE19855649**.**

Comme exemples de filtres organiques, on peut citer désignés ci-dessus sous leur nom INCI :
Dérivés de l'acide para-aminobenzoipue :
   - PABA,
   - Ethyl PABA,
   - Ethyl Dihydroxypropyl PABA,
   - Ethylhexyl Diméthyl PABA vendu notamment sous le nom « ESCALOL 507 » par ISP,
   - Glyceryl PABA,
   - PEG-25 PABA vendu sous le nom « UVINUL P25 » par BASF,
Dérivés salicyliques :
   - Homosalate vendu sous le nom « EUSOLEX HMS » par RONA/EM INDUSTRIES,
   - Ethylhexyl Salicylate vendu sous le nom « NEO HELIOPAN OS » par HAARMANN et REIMER,
   - Dipropyleneglycol Salicylate vendu sous le nom « DIPSAL » par SCHER,
   - TEA Salicylate, vendu sous le nom « NEO HELIOPAN TS » par HAARMANN et REIMER,
Dérivés du dibenzoylméthane :
   - Butyl Methoxydibenzoylmethane vendu notamment sous le nom commercial « PARSOL 1789 » par HOFFMANN LA ROCHE,
   - lsopropyl Dibenzoylmethane,
Dérivés de β,β'-diphénylacrylate :
   - Octocrylene vendu notamment sous le nom commercial « UVINUL N539 » par BASF,
   - Etocrylene, vendu notamment sous le nom commercial « UVINUL N35 » par BASF,
Dérivés de la benzophénone :
   - Benzophenone-1 vendu sous le nom commercial « UVINUL 400 » par BASF,
   - Benzophenone-2 vendu sous le nom commercial « UVINUL D50 » par BASF
   - Benzophenone-3 ou Oxybenzone, vendu sous le nom commercial « UVINUL M40 » par BASF,
   - Benzophenone-4 vendu sous le nom commercial « UVINUL MS40 » par BASF,
   - Benzophenone-5
   - Benzophenone-6 vendu sous le nom commercial «HELISORB 11» par NORQUAY
   - Benzophenone-8 vendu sous le nom commercial « SPECTRA-SORB UV-24 » PAR AMERICAN CYANAMID
   - Benzophenone-9 vendu sous le nom commercial« UVINUL DS-49» par BASF,
   - Benzophenone-12
Dérivés du benzylidène camphre :
   - 3-Benzylidene camphor fabriqué sous le nom « MEXORYL SD» par CHIMEX,
   - 4-Methylbenzylidene camphor vendu sous le nom « EUSOLEX 6300 » par MERCK,
   - Benzylidene Camphor Sulfonic Acid fabriqué sous le nom « MEXORYL SL» par CHIMEX,
   - Camphor Benzalkonium Methosulfate fabriqué sous le nom « MEXORYL SO » par CHIMEX,
   - -Terephthalylidene Dicamphor Sulfonic Acid fabriqué sous le nom « MEXORYL SX » par CHIMEX,
   - Polyacrylamidomethyl Benzylidene Camphor fabriqué sous le nom « MEXORYL SW » par CHIMEX,
Dérivés du phenyl benzimidazole :
   - Phenylbenzimidazole Sulfonic Acid vendu notamment sous le nom commercial « EUSOLEX 232 » par MERCK,
   - Disodium Phenyl Dibenzimidazole Tetra-sulfonate vendu sous le nom commercial commercial « NEO HELIOPAN AP » par HAARMANN et REIMER,
Dérivés de triazine :
   - Anisotriazine vendu sous le nom commercial «TINOSORB S » par CIBA SPECIALTY CHEMICALS
   - Ethylhexyl triazone vendu notamment sous le nom commercial «UVINUL T150» par BASF,
   - Diethylhexyl Butamido Triazone vendu sous le nom commercial « UVASORB HEB » par SIGMA 3V,
   - la 2,4,6-tris-(4'-amino benzalmalonate de diisobutyle)-s- triazine.
Dérivés du phenyl benzotriazole :
   - Drometrizole Trisiloxane vendu sous le nom « SILATRIZOLE » par RHODIA CHIMIE ,
   - Methylène bis-Benzotriazolyl Tetramethylbutylphénol, vendu sous forme solide sous le nom commercial « MIXXIM BB/100 » par FAIRMOUNT CHEMICAL ou sous forme micronisé en dispersion aqueuse sous le nom commercial « TINOSORB M » par CIBA SPECIALTY CHEMICALS,
Dérivés anthraniliques :
   - Menthyl anthranilate vendu sous le nom commercial commercial « NEO HELIOPAN MA » par HAARMANN et REIMER,
Dérivés d'imidazolines :
   - Ethylhexyl Dimethoxybenzylidene Dioxoimidazoline Propionate,
Dérivés du benzalmalonate :
   - Polyorganosiloxane à fonctions benzalmalonate vendu sous la dénomination commerciale « PARSOL SLX » par HOFFMANN LA ROCHE
et leurs mélanges.

Les filtres UV organiques plus particulièrement préférés sont choisis parmi les composés suivants :
- Ethylhexyl Salicylate,
- Octocrylene,
- Phenylbenzimidazole Sulfonic Acid,
- Terephthalylidene Dicamphor Sulfonic,
- Benzophenone-3,
- Benzophenone-4,
- Benzophenone-5,
- 4-Methylbenzylidene camphor,
- Disodium Phenyl Dibenzimidazole Tetra-sulfonate
- Anisotriazine,
- Ethylhexyl triazone,
- Diethylhexyl Butamido Triazone,
- la 2,4,6-tris-(4'-amino benzalmalonate de diisobutyle)-s- triazine
- Methylene bis-Benzotriazolyl Tetramethylbutylphenol,
- Drometrizole Trisiloxane,
et leurs mélanges.

Les compositions cosmétiques selon l'invention peuvent encore contenir des pigments ou bien encore des nanopigments (taille moyenne des particules primaires: généralement entre 5 nm et 100 nm, de préférence entre 10 nm et 50 nm) d'oxydes métalliques enrobés ou non comme par exemple des nanopigments d'oxyde de titane (amorphe ou cristallisé sous forme rutile et/ou anatase), de fer, de zinc, de zirconium ou de cérium qui sont tous des agents photoprotecteurs UV bien connus en soi. Des agents d'enrobage classiques sont par ailleurs l'alumine et/ou le stéarate d'aluminium. De tels nanopigments d'oxydes métalliques, enrobés ou non enrobés, sont en particulier décrits dans les demandes de brevets EP-A-0518772 et EP-A-0518773.

Les compositions selon l'invention peuvent également contenir des agents de bronzage et/ou de brunissage artificiels de la peau (agents autobronzants), tels que par exemple de la dihydroxyacétone (DHA).

Les compositions de l'invention peuvent comprendre en outre des adjuvants cosmétiques classiques notamment choisis parmi les corps gras, les solvants organiques, les épaississants ioniques ou non ioniques, les adoucissants, les antioxydants, les agents anti radicaux libres, les opacifiants, les stabilisants, les émollients, les silicones, les α-hydroxyacides, les agents anti-mousse, les agents hydratants, les vitamines, les agents répulsifs contre les insectes, les parfums, les conservateurs, les tensioactifs, les anti-inflammatoires, les antagonistes de substance P, les charges, les polymères, les propulseurs, les agents alcalinisants ou acidifiants, les colorants ou tout autre ingrédient habituellement utilisé en cosmétique, en particulier pour la fabrication de compositions antisolaires sous forme d'émulsions.

Les corps gras peuvent être constitués par une huile ou une cire ou leurs mélanges. Par huile, on entend un composé liquide à température ambiante. Par cire, on entend un composé solide ou substantiellement solide à température ambiante, et dont le point de fusion est généralement supérieur à 35°C. Ils comprennent également les acides gras, les alcools gras et les esters d'acides gras, linéaires ou cycliques tels que les dérivés d'acide benzoïque, trimellitique et hydroxy-benzoïque.

Comme huiles, on peut citer les huiles minérales (paraffine); végétales (huile d'amande douce, de macadamia, de pépin de cassis, de jojoba) ; synthétiques comme le perhydrosqualène, les alcools, les acides ou les esters gras (comme le benzoate d'alcools en C₁₂-C₁₅ vendu sous la dénomination commerciale « Finsolv TN » par la société Finetex, le palmitate d'octyle, le lanolate d'isopropyle, les triglycérides dont ceux des acides caprique/caprylique), les esters et éthers gras oxyéthylénés ou oxypropylénés; siliconées (cyclométhicone, polydiméthysiloxanes ou PDMS) ou fluorées, les polyalkylènes.

Comme composés cireux, on peut citer la paraffine, la cire de carnauba, la cire d'abeille, l'huile de ricin hydrogénée.

Parmi les solvants organiques, on peut citer les alcools et polyols inférieurs.

Bien entendu, l'homme de l'art veillera à choisir ce ou ces éventuels composés complémentaires et/ou leurs quantités de manière telle que les propriétés avantageuses, en particulier la photostabilité, attachées intrinsèquement aux compositions conformes à l'invention ne soient pas, ou substantiellement pas, altérées par la ou les adjonctions envisagées.

Les compositions de l'invention peuvent être préparées selon les techniques bien connues de l'homme de l'art, en particulier celles destinées à la préparation d'émulsions de type huile-dans-eau ou eau-dans-huile.

Ces compositions peuvent se présenter en particulier sous forme d'émulsion, simple ou complexe (H/E, E/H, H/E/H ou E/H/E) telle qu'une crème, un lait, un gel ou un gel crème, de poudre, de bâtonnet solide et éventuellement être conditionnée en aérosol et se présenter sous forme de mousse ou de spray.

Lorsqu'il s'agit d'une émulsion, la phase aqueuse de celle-ci peut comprendre une dispersion vésiculaire non ionique préparée selon des procédés connus (Bangham, Standish and Watkins. J. Mol. Biol. 13, 238 (1965), FR2315991 et FR2416008).

La composition cosmétique de l'invention peut être utilisée comme composition protectrice de l'épiderme humain ou des cheveux contre les rayons ultraviolets, comme composition antisolaire ou comme produit de maquillage.

Lorsque la composition cosmétique selon l'invention est utilisée pour la protection de l'épiderme humain contre les rayons UV, ou comme composition antisolaire, elle peut se présenter sous forme de suspension ou de dispersion dans des solvants ou des corps gras, sous forme de dispersion vésiculaire non ionique ou encore sous forme d'émulsion, de préférence de type huile-dans-eau, telle qu'une crème ou un lait, sous forme de pommade, de gel, de gel crème, de bâtonnet solide, de poudre, de stick, de mousse aérosol ou de spray.

Lorsque la composition cosmétique selon l'invention est utilisée pour la protection des cheveux contre les rayons UV, elle peut se présenter sous forme de shampooing, de lotion, de gel, d'émulsion, de dispersion vésiculaire non ionique et constituer par exemple une composition à rincer, à appliquer avant ou après shampooing, avant ou après coloration ou décoloration, avant, pendant ou après permanente ou défrisage, une lotion ou un gel coiffants ou traitants, une lotion ou un gel pour le brushing ou la mise en plis, une composition de permanente ou de défrisage, de coloration ou décoloration des cheveux.

Lorsque la composition est utilisée comme produit de maquillage des cils, des sourcils ou de la peau, tel que crème de traitement de l'épiderme, fond de teint, bâton de rouge à lèvres, fard à paupières, fard à joues, mascara ou ligneur encore appelé "eye liner', elle peut se présenter sous forme solide ou pâteuse, anhydre ou aqueuse, comme des émulsions huile dans eau ou eau dans huile, des dispersions vésiculaires non ioniques ou encore des suspensions.

A titre indicatif, pour les formulations antisolaires conformes à l'invention qui présentent un support de type émulsion huile-dans-eau, la phase aqueuse (comprenant notamment les filtres hydrophiles) représente généralement de 50 à 95% en poids, de préférence de 70 à 90% en poids, par rapport à l'ensemble de la formulation, la phase huileuse (comprenant notamment les filtres lipophiles) de 5 à 50% en poids, de préférence de 10 à 30% en poids, par rapport à l'ensemble de la formulation, et le ou les (co)émulsionnant(s) de 0,5 à 20% en poids, de préférence de 2 à 10% en poids, par rapport à l'ensemble de la formulation.

Comme indiqué en début de description, un objet de l'invention est l'utilisation d'une composition telle que définie précédemment pour la fabrication d'une composition cosmétique ou dermatologique destinées à la protection de la peau et/ou des cheveux contre le rayonnement ultraviolet, en particulier le rayonnement solaire.

Un autre objet de la présente invention réside dans un procédé pour améliorer la stabilité d'au moins un dérivé du dibenzoylméthane vis-à-vis du rayonnement UV consistant à associer au dit dérivé de dibenzoylméthane une quantité efficace d'au moins un 4,4-diarylbutadiène tel que défini ci-dessus.

Un autre objet de la présente invention consiste en l'utilisation d'un filtre UV du type 4,4-diarylbutadiène tel que défini ci-dessus dans une composition cosmétique comprenant au moins un filtre UV du type dérivé du dibenzoylméthane dans le but de d'améliorer la stabilité vis-à-vis des rayons UV dudit dérivé du dibenzoylméthane ; **le rapport en poids du composé 4,4-diarylbutadiène sur le dérivé de dibenzoylméthane étant supérieur à 2,5 et ladite composition ne contenant pas de dérivé de cinnamate.**

Des exemples concrets, mais nullement limitatifs, illustrant l'invention, vont maintenant être donnés.

| **COMPOSITION** | **EX 1** |
|---|---|
| Mélange mono /distéarate de glycerol /stéarate de | 2 |
| polyéthylène glycol (100 OE) (ARLACEL 165 FL - ICI) | |
| Alcool stéarylique | 1 |
| (LANETTE 18 - HENKEL) | |
| Acide stéarique d'huile de palme | 2.5 |
| (STEARINE TP - STEARINERIE DUBOIS) | |
| poly diméthylsiloxane | 0.5 |
| (DOW CORNING 200 FLUID - DOW CORNING) | |
| Benzoate d'alcools en C12/C15 | 20 |
| (WITCONOL TN -WITCO) | |
| Triéthanolamine | 0.5 |
| Butyl Methoxydibenzoylmethane | 2 |
| (PARSOL 1789 - HOFFMANN LA ROCHE) | |
| Composé de formule (1) | 8 |
| Glycérine | 4 |
| Triéthanolamine | 0.3 |
| Acide polyacrylique | 0.4 |
| (SYNTHALEN K - 3V) | |
| Conservateurs | qs |
| Eau démineralisée qsp | 100 g |

| **COMPOSITION** | **EX 2** |
|---|---|
| Mélange mono /distéarate de glycerol / stéarate de | 2 |
| polyéthylène glycol (100 OE) (ARLACEL 165 FL - ICI) | |
| Alcool stéarylique | 1 |
| (LANETTE 18 - HENKEL) | |
| Acide stéarique d'huile de palme | 2.5 |
| (STEARINE TP - STEARINERIE DUBOIS) | |
| Polydiméthylsiloxane | 0.5 |
| (DOW CORNING 200 FLUID - DOW CORNING) | |
| Benzoate d'alcools en C12/C15 | 20 |
| (WITCONOL TN -WITCO) | |
| Triéthanolamine | 0.5 |
| Butyl Methoxydibenzoylmethane | 2 |
| (PARSOL 1789 HOFFMANN LA ROCHE) | |
| Composé de formule (6) | 8 |
| Glycérine | 4 |
| Triéthanolamine | 0.3 |
| Acide polyacrylique | 0.4 |
| (SYNTHALEN K - 3V) | |
| Conservateurs | qs |
| Eau démineralisée qsp | 100 g |

## Revendications

1. Composition cosmétique ou dermatologique, à usage topique, **caractérisée par le fait qu'**elle comprend, dans un support cosmétiquement acceptable :
(a) au moins un filtre UV du type dérivé du dibenzoylméthane et
(b) au moins un composé 4,4-diarylbutadiène; le rapport en poids du composé 4.4-diarylbutadiène sur le dérivé de dibenzoylméthane étant supérieur à 2,5 et ladite composition ne contenant pas de dérivé de cinnamate.

2. Composition selon la revendication 1, où le composé 4,4-diarylbutadiène répond à la formule (I) suivante : 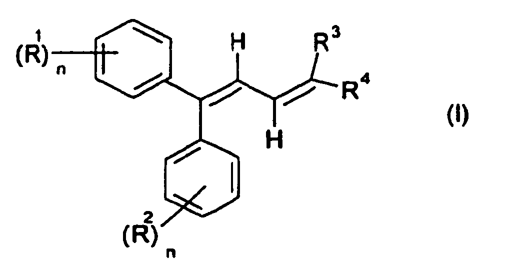 dans laquelle le système diène est de configuration Z,Z ; Z,E ; E,Z ou E,E ou des mélanges desdites configurations et où :
- R¹ et R², identiques ou différents, désignent hydrogène, un radical alkyle en C₁-C_{20.} linéaire ou ramifié : un radical alcényle en C₂- C₁₀; un radical alcoxy en C₁-C₁₂; un radical cycloalkyle en C₃-C₁₀ ; un radical cycloalcényle en C₃-C₁₀; un radical alcoxycarbonyle en C₁-C₂₀ linéaire ou ramifié ; un radical monoalkylamino en C₁-C₁₂, linéaire ou ramifié ; un radical dialkylamino en C₁-C₁₂ linéaire ou ramifié : un aryle; un hétéroaryle ou un substituant hydrosolubilisant choisi parmi un groupe carboxylate, un groupe sulfonate ou un reste ammonium ;
- R désigne un groupe COOR⁵; COR⁵; CONR⁵R⁶; CN ; un radical alkyle en C₁-C₂₀. linéaire ou ramifié ; un radical alcényle en C₂- C₁₀ ; un radical cycloalkyle en C₃-C₁₀ ; un radical bicycloalkyle en C₇-C₁₀; un radical cycloalcényle en C₃-C₁₀; un radical bicycloalcényle en C₇-C₁₀ : un aryle en C₆-C₁₈ ; un hétéroaryle en C₃-C₇;
- R⁴ désigne un groupe COOR⁶; COR⁶; CONR⁵R⁶ ; CN ; un radical alkyle en C₁-C_{20.} linéaire ou ramifié ; un radical alcényle en C₂- C₁₀; un radical cycloalkyle en C₃-C₁₀; un radical bicycloalkyle en C₇-C₁₀; un radical cycloalcényle en C₃-C₁₀ ; ; un radical bycloalcényle en C₇-C₁₀ ; un aryle ; un hétéroaryle ;
- R⁵ et R⁶ identiques ou différents, désignent hydrogène ; [X]ₚ-R⁷, C₁-C₆-alkylène- . SO₃Y; C₁-C₆-alkylène-PO₃Y; C₁-C₆-alkylène-N(R⁸)₃⁺ A⁻; un radical alkyle en C₁-C₂₀, linéaire ou ramifié ; un radical alcényle en C₂-C₁₀; un radical cycloalkyle en C₃-C₁₀; un radical bicycloalkyle en C₇-C₁₀; un radical cycloalcényle en C₃-C₁₀ ; un radical bicycloalcényle en C₇-C₁₀ ; un aryle ; un hétéroaryle ;
- X désigne un groupe -CH₂-CH₂-Z-, -CH₂CH₂CH₂Z-. -CH(CH₃)-CH₂-Z-, -CH₂-CH₂-CH₂-CH₂-Z-, -CH₂-CH(CH₂CH₃)-Z- ;
- A désigne CI, Br, I, SO₄R⁹ ;
- Y désigne hydrogène, Na⁺, K⁺, Mg²⁺, Ca²⁺, Li⁺, Al³⁺, -N(R⁸)₄⁺
- Z désigne O ou NH ;
- R⁷ et R⁸ identiques ou différents, désignent hydrogène, un radical alkyle en C₁-C₆, linéaire ou ramifié ; un radical alcényle en C₂- C₆, linéaire ou ramifié ; un radical acyle en C₁-C₆ linéaire ou ramifié ;
- R⁹ désigne hydrogène, un radical alkyle en C₁-C₆, linéaire ou ramifié ; un radical alcényle en C₂- C₆ ;
- n varie de1 à 3;
- p varie de 0 à 150.

3. Composition selon la revendication 2, où le composé de formule (1) est choisi parmi ceux de formule (la) suivante : 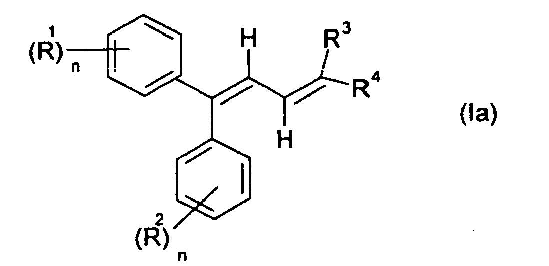 dans laquelle le système diène est de configuration Z,Z ; Z,E ; E,Z ou E,E ou des mélanges desdites configurations et où :
- R¹ et R², identiques ou différents, désignent hydrogène, un radical alkyle en C₁-C₈ ; un radical alcoxy en C₁-C₈ ; un substituant hydrosolubilisant choisi parmi un groupe carboxylate, un groupe sulfonate ou un reste ammonium ;
- R désigne un groupe COOR⁵ ; CONR⁵R⁶ ; CN ;
- R⁴ désigne un groupe COOR⁶ ; CONR⁵R⁶ ;
- R⁵ désigne hydrogène : [X]ₚ-R⁷ ; C₁-C₆-alkylène-SO₃Y; C₁-C₆-alkylène-N(R⁸)₃⁺ A- ;
- R⁶ désigne [X]ₚ-R⁷ ; C₁-C₆-alkylène-SO₃Y : C₁-C₆-alkylène-N(R⁸)₃⁺ A- ;
- X désigne un groupe -CH₂-CH₂-O-, -CH₂CH₂CH₂O-, -CH(CH₃)-CH₂-O-,
- A désigne CI, Br, I, SO₄R⁹ ;
- Y désigne hydrogène. Na⁺, K⁺, Mg²⁺, Ca²⁺, Li⁺, Al³⁺, -N(R⁸)₄⁺
- R⁷, R⁸ et R identiques ou différents, désignent hydrogène, un radical alkyle en C₁-C₃, linéaire ou ramifié ;
- n varie de 1 à 3
- p varie de 0 à 50.

4. Composition selon la revendication 3, où le composé de formule (I) est choisi parmi ceux de formule formule (lb) suivante : 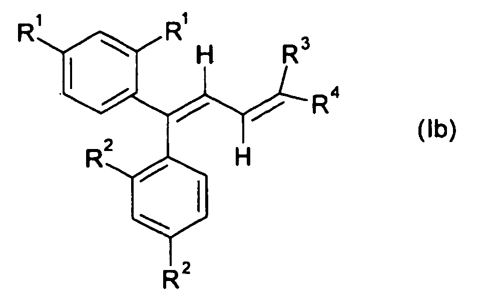 dans laquelle le système diène est de configuration Z,Z ; Z,E ; E,Z ou E,E ou des mélanges desdites configurations et où :
- R¹ et R², identiques ou différents, désignent hydrogène, un radical alkyle en C₁-C₈ ; un radical alcoxy en C₁-C₈;
- R³ désigne un groupe COOR⁵; CONR⁵R⁶; CN ;
- R⁴ désigne un groupe COOR⁶ ; CONR⁵R⁶;
- R⁵ désigne hydrogène ; [X]ₚ-R⁷; C₁-C₆-alkylène-SO₃Y; C₁-C₆-alkylène-N(R⁸)₃+ A⁻ ;
- R⁶ désigne [X]ₚ-R⁷; C₁-C₆-alkylène-SO₃Y; C₁-C₆-alkylène-N(R⁸)3⁺ A⁻;
- X désigne un groupe -CH₂-CH₂-O-, -CH₂CH₂CH₂O-, -CH(CH₃)-CH₂-O-,
- A désigne CI, Br, I, SO₄R⁹;
- Y désigne hydrogène, Na⁺, K⁺, Mg²⁺, Ca²⁺, Li⁺, Al³⁺, -N(R⁸)₄⁺
- R⁷ , R⁸ et R⁹ identiques ou différents, désignent hydrogène, un radical alkyle en C₁-C₃, linéaire ou ramifié ;
- p varie de 0 à 50.

5. Composition selon la revendication 4, où le composé de formule (I) est choisi parmi ceux répondant à la formule (Ic) suivante : 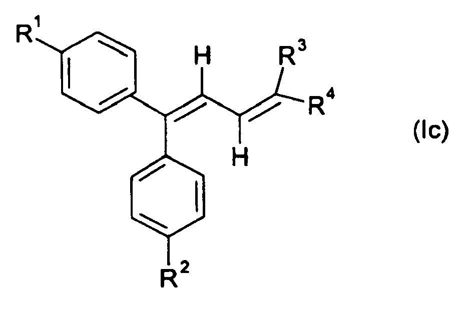 dans laquelle le système diène est de configuration Z,Z ; Z,E ; E,Z ou E,E ou des mélanges desdites configurations et où :
- R¹ et R² identiques ou différents, désignent hydrogène, un radical alkyle en C₁-C₈; un radical alcoxy en C₁-C₈;
- R³ désigne un groupe COOR⁵; CONR⁵R⁶; CN ;
- R⁴ désigne un groupe COOR⁶ ; CONR⁵R⁶;
- R⁵ désigne hydrogène ; [X]ₚ-R⁷ ; C₁-C₆-alkylène-SO₃Y; C₁-C₆-alkylène-N(R⁸)₃⁺ A⁻ ;
- R⁶ désigne [X]ₚ-R⁷ ; C₁-C₆-alkylène-SO₃Y ; C₁-C₆-alkylène-N(R⁸)₃⁺ A⁻ ;
- X désigne un groupe -CH₂-CH₂-O-, -CH₂CH₂CH₂O-, -CH(CH₃)-CH₂-O
- A désigne CI, Br, l, SO₄R⁹ - Mg²⁺, Al³⁺,
- Y désigne hydrogène, Na⁺, K⁺, Mg²⁺, Ca²⁺, Li⁺, Al³⁺, -N(R⁸)₄⁺
- R⁷, R⁸ et R identiques ou différents, désignent hydrogène, un radical alkyle en C₁-C₃, linéaire ou ramifié ;
- p varie de 0 à 50.

6. Composition selon la revendication 4 ou 5, où le composé de formule (I) est choisi parmi les composés suivants :

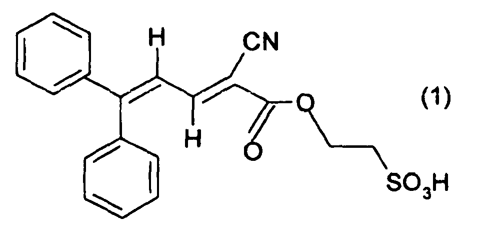

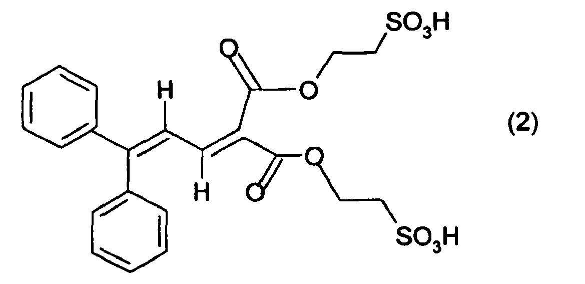

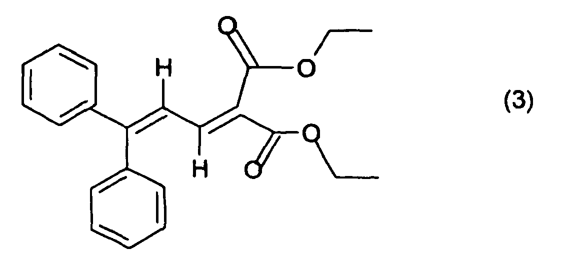

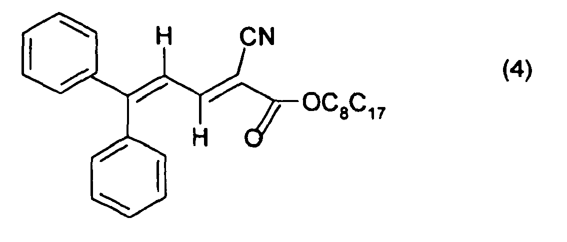

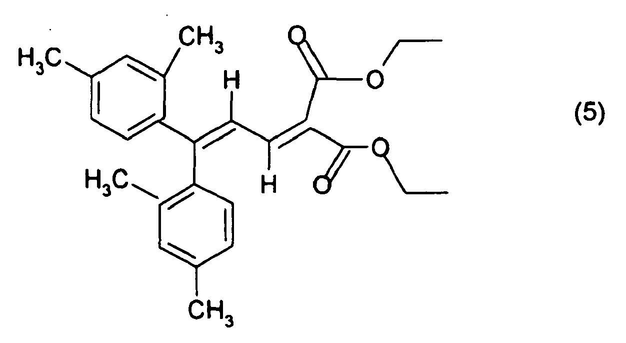

7. Composition selon la revendication 1, où le composé 4,4-diarylbutadiène est un oligomère répondant à la formule (II) suivante : 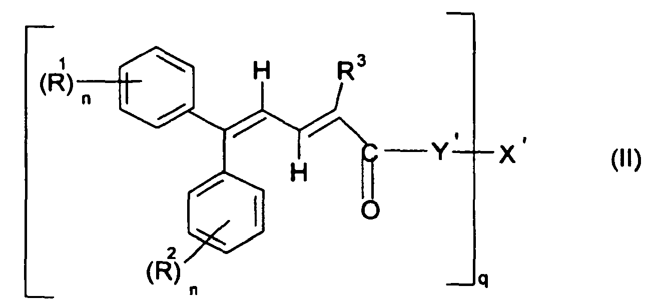 dans laquelle le système diène est de configuration Z,Z ; Z,E ; E,Z ou E,E ou des mélanges desdites configurations et où :
- R¹, R² R³ et n ont les mêmes significations indiquées dans la formule (I) dans la revendication 2 ;
- Y' désigne un groupe -O- ou -NR¹⁰-
- R¹⁰ désigne hydrogène ; un radical alkyle en C₁-C₂₀, linéaire ou ramifié ; un radical alcényle en C₂- C₁₀; un radical cycloalkyle en C₃-C₁₀; un radical bicycloalkyle en C₇-C₁₀; un radical cycloalcényle en C₃-C₁₀; ; un radical bicycloalcényle en C₇-C₁₀ ; un aryle ; un hétéroaryle ;
- X' désigne un reste de polyol linéaire ou ramifié, aliphatique ou cycloaliphatique comprenant de 2 à 10 groupes hydroxy et de valence q ; la chaîne carbonée dudit reste pouvant être interrompue par un ou plusieurs atomes de souffre ou d'oxygène ; un ou plusieurs groupes imines ou un ou plusieurs alkylimino en C₁-C₄ ;
- q varie de 2 à 10.

8. Composition selon la revendication 7, où le composé de formule (II) est choisi parmi ceux pour lesquels :
- R¹ et R², identiques ou différents, désignent hydrogène, un radical alkyle en C₁-C₁₂; un radical alcoxy en C₁-C₈ ; un substituant hydrosolubilisant choisi parmi un groupe carboxylate, un groupe sulfonate ou un reste ammonium ;
- R³ désigne un groupe COOR⁵; CONR⁵R⁶; CN ; un radical cycloalkyle en C₃-C₁₀ ; un radical bicycloalkyle en C₇-C₁₀ ;
- R⁵ et R⁶ , identiques ou différents, désignent un radical alkyle en C₁-C₂₀, linéaire ou ramifié ; un radical cycloalkyle en C₃-C₁₀ ; un radical bicycloalkyle en C₇-C₁₀ ; naphtyle ou phényle éventuellement substitué ;
- X' désigne un reste de polyol comprenant de 2 à 6 groupes hydroxy et plus particulièrement de 2 à 4.

9. Composition selon la revendication 8, où le composé de formule (II) est choisi parmi ceux pour lesquels :
- X' désigne un reste d'éthanol ou de pentaérythrol.

10. Composition selon la revendication 9, où le composé de formule (II) est choisi parmi les composés suivants :

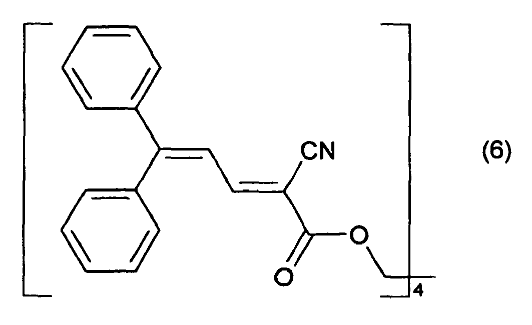

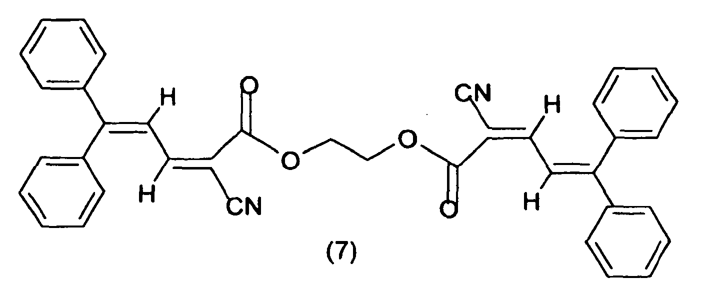

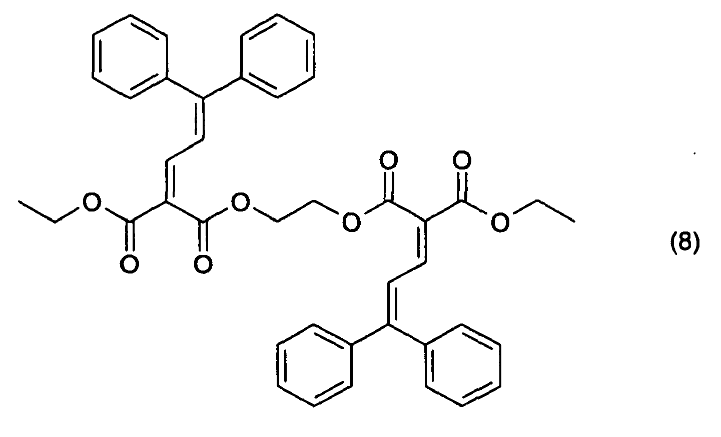

11. Composition selon l'une quelconque des revendications 1 à 10, **caractérisée par le fait que** le dérivé de dibenzoylméthane est choisi parmi :
- le 2-méthyldibenzoylméthane
- le 4-méthyldibenzoylméthane
- le 4-isopropyldibenzoylméthane
- le 4-tert.-butyldibenzoylméthane
- le 2,4-diméthyldibenzoylméthane
- le 2,5-diméthyldibenzoylméthane
- le 4,4'-diisopropyldibenzoylméthane
- le 4,4'-diméthoxydibenzoylméthane
- le 4-tert.-butyl-4'-méthoxydibenzoylméthane
- le 2-méthyl-5-isopropyl-4'-méthoxydibenzoylméthane
- le 2-méthyl-5-tert.-butyl-4'-méthoxydibenzoylméthane
- le 2,4-diméthyl-4'-méthoxydibenzoylméthane
- le 2,6-diméthyl-4-tert.-butyl-4'-méthoxydibenzoylméthane.

12. Composition selon la revendication 11, **caractérisée par le fait que** le dérivé de dibenzoylméthane est le 4-(ter.-butyl) 4'-méthoxy dibenzoylméthane.

13. Composition selon la revendication 12, **caractérisée par le fait que** le dérivé de dibenzoylméthane est le 4-isopropyl-dibenzoylméthane.

14. Composition selon l'une quelconque des revendications 1 à 13, où le composé 4,4-diarylbutadiène est présent à des teneurs allant de 0,5 % à 15 % en poids, par rapport au poids total de la composition.

15. Composition selon la revendication 14, où le composé 4,4-diarylbutadiène est présent à des teneurs allant de 1 % à 10% en poids, par rapport au poids total de la composition.

16. Composition selon l'une quelconque des revendications 1 à 14, où le dérivé du dibenzoylméthane est présent à des teneurs allant de 0,5 % à 15 % en poids, par rapport au poids total de la composition.

17. Composition selon la revendication 16, où le dérivé du dibenzoylméthane est présent à des teneurs allant de 1 % à 10% en poids, par rapport au poids total de la composition.

18. Composition selon l'une quelconque des revendications 1 à 17, **caractérisée par le fait qu'**elle contient en plus d'autres filtres organiques actifs dans l'uV-A et/ou l'UV-B.

19. Composition selon la revendication 16, où le ou les filtres UV organiques complémentaires sont choisis parmi les anthranilates ; les dérivés salicyliques, les dérivés du camphre ; les dérivés de la benzophénone ; les dérivés de β,β'-diphénylacrylate, les dérivés de benzotriazole ; les dérivés de benzalmalonate ; les dérivés de benzimidazole ; les imidazolines ; les dérivés bis-benzoazolyle ; les dérivés de l'acide p-aminobenzoïque (PABA) ; les dérivés de méthylène bis-(hydroxyphényl benzotriazole) ; les polymères filtres et silicones filtres ; les dimères dérivés d'α-alkylstyrène.

20. Composition selon la revendication 19, **caractérisée par le fait que** le ou les filtres UV organiques sont choisis parmi les composés suivants :
- Ethylhexyl Salicylate,
- Octocrylene,
- Phenylbenzimidazole Sulfonic Acid,
- Terephthalylidene Dicamphor Sulfonic Acid,
- Benzophenone-3,
- Benzophenone-4,
- Benzophenone-5,
- 4-Methylbenzylidene camphor,
- Disodium Phenyl Dibenzimidazole Tetra-sulfonate
- Anisotriazine,
- Ethylhexyl triazone,
- Diethylhexyl Butamido Triazone,
- la 2,4,6-tris-(4'-amino benzalmalonate de diisobutyle)-s- triazine
- Methylene bis-Benzotriazolyl Tetramethylbutylphenol,
- Drometrizole Trisiloxane,
et leurs mélanges.

21. Composition selon l'une quelconque des revendications 1 à 20, **caractérisée par le fait qu'**elle comprend en outre, des pigments ou des nanopigments d'oxydes métalliques, enrobés ou non.

22. Composition selon la revendication 19, **caractérisée par le fait que** lesdits pigments ou nanopigments sont choisis parmi les oxydes de titane, de zinc, de fer, de zirconium, de cérium et leurs mélanges; enrobés ou non.

23. Composition selon l'une quelconque des revendications 1 à 22, **caractérisée par le fait qu'**elle comprend en outre au moins un agent de bronzage et/ou de brunissage artificiel de la peau.

24. Composition selon l'une quelconque des revendications 1 à 23, **caractérisée par le fait qu'**elle comprend en outre au moins un adjuvant choisi parmi les corps gras, les solvants organiques, les épaississants ioniques ou non ioniques, les adoucissants, les antioxydants, les agents anti radicaux libres, les opacifiants, les stabilisants, les émollients, les silicones, les α-hydroxyacides, les agents anti-mousse, les agents hydratants, les vitamines, les agents répulsifs contre les insectes, les parfums, les conservateurs, les tensioactifs, les anti-inflammatoires, les antagonistes de substance P, les charges, les polymères, les propulseurs, les agents alcalinisants ou acidifiants, les colorants.

25. Composition selon l'une quelconque des revendications 1 à 24, **caractérisée par le fait qu'**il s'agit d'une composition protectrice de l'épiderme humain ou d'une composition antisolaire et qu'elle se présente sous forme d'une dispersion vésiculaire non ionique, d'une émulsion, en particulier d'une émulsion de type huile-dans-eau, d'une crème, d'un lait, d'un gel, d'un gel crème, d'une suspension, d'une dispersion, d'une poudre, d'un bâtonnet solide, d'une mousse ou d'un spray.

26. Composition selon l'une quelconque des revendications 1 à 24, **caractérisée par le fait qu'**il s'agit d'une composition de maquillage des cils, des sourcils ou de la peau et qu'elle se présente sous forme solide ou pâteuse, anhydre ou aqueuse, d'une émulsion, d'une suspension ou d'une dispersion.

27. Composition selon l'une quelconque des revendications 1 à 24, **caractérisée par le fait qu'**il s'agit d'une composition destinée à la protection des cheveux contre les rayons ultraviolets et qu'elle se présente sous la forme d'un shampooing, d'une lotion, d'un gel, d'une émulsion, d'une dispersion vésiculaire non ionique.

28. Utilisation d'un composé 4,4-diarylbutadiène tel que défini dans l'une quelconque des revendications précédentes dans une composition cosmétique comprenant au moins un filtre UV du type dérivé du dibenzoylméthane dans le but de d'améliorer la stabilité vis-à-vis des rayons UV dudit dérivé du dibenzoylméthane ; **le rapport en poids du composé 4,4-diarylbutadiène sur le dérivé de dibenzoylméthane étant supérieur à 2,5 et ladite composition ne contenant pas de dérivé de cinnamate.**

## Revendications (Revendications pour l'(les) Etat(s) contractant(s) suivant(s): DE)

1. Composition cosmétique ou dermatologique, à usage topique, **pour la protection de l'épiderme humain contre les rayons UV, caractérisée par le fait qu'**elle comprend, dans un support cosmétiquement acceptable :
(a) au moins un filtre UV du type dérivé du dibenzoylméthane et
(b) au moins un composé 4,4-diarylbutadiène ; le rapport en poids du composé 4,4-diarylbutadiène sur le dérivé de dibenzoylméthane étant supérieur à 2,5 et ladite composition ne contenant pas de dérivé de cinnamate et qu'elle se présente sous forme de dispersion vésiculaire non ionique ; sous forme d'émulsion huile/eau; sous forme de gel; sous forme de poudre ; sous forme de bâtonnet solide ; sous forme de mousse aérosol ou de spray.

2. Composition cosmétique ou dermatologique, à usage topique, **pour la protection des cheveux contre les rayons UV**, **caractérisée par le fait qu'**elle comprend, dans un support cosmétiquement acceptable :
(a) au moins un filtre UV du type dérivé du dibenzoylméthane et
(b) au moins un composé 4,4-diarylbutadiène; le rapport en poids du composé 4,4-diarylbutadiène sur le dérivé de dibenzoylméthane étant supérieur à 2,5 et ladite composition ne contenant pas de dérivé de cinnamate et qu'elle se présente sous forme de shampooing, de gel ou de dispersion vésiculaire non ionique.

3. Composition **de maquillage des cils, des sourcils ou de la peau, caractérisée par le fait qu'**elle comprend, dans un support cosmétiquement acceptable :
(a) au moins un filtre UV du type dérivé du dibenzoylméthane et
(b) au moins un composé 4,4-diarylbutadiène ; le rapport en poids du composé 4,4-diarylbutadiène sur le dérivé de dibenzoylméthane étant supérieur à 2,5 et ladite composition ne contenant pas de dérivé de cinnamate **et qu'elle se présente sous forme solide ou pâteuse, anhydre ou aqueuse sous forme d'émulsion huile/eau ou de dispersion vésiculaire non ionique.**

4. Composition **selon l'une quelconque des revendications 1 à 3,** où le composé 4,4-diarylbutadiène répond à la formule (I) suivante : 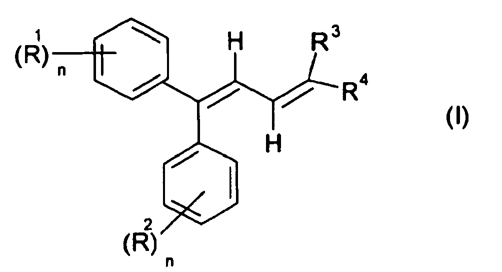 dans laquelle le système diène est de configuration Z,Z ; Z,E ; E,Z ou E,E ou des mélanges desdites configurations et où :
- R¹ et R², identiques ou différents, désignent hydrogène, un radical alkyle en C₁-C₂₀, linéaire ou ramifié : un radical alcényte en C₂- C₁₀ ; un radical alcoxy en C₁-C₁₂ ; un radical cycloalkyle en C₃-C₁₀ ; un radical cycloalcényle en C₃-C₁₀ ; un radical alcoxycarbonyle en C₁-C₂₀ linéaire ou ramifié ; un radical monoalkylamino en C₁-C₁₂, linéaire ou ramifié ; un radical dialkylamino en C₁-C₁₂, linéaire ou ramifié ; un aryle; un hétéroaryle ou un substituant hydrosolubilisant choisi parmi un groupe carboxylate, un groupe sulfonate ou un reste ammonium ;
- R désigne un groupe COOR⁵ ; COR⁵; CONR⁵R⁶; CN ; un radical alkyle en C₁-C_{20.} linéaire ou ramifié ; un radical alcényle en C₂- C₁₀ ; un radical cycloalkyle en C₃-C₁₀ ; un radical bicycloalkyle en C₇-C₁₀ ; un radical cycloalcényle en C₃-C₁₀ ; un radical bicycloalcényle en C₇-C₁₀ ; un aryle en C₆-C₁₈ ; un hétéroaryle en C₃-C₇ ;
- R⁴ désigne un groupe COOR⁸ ; COR⁶; CONR⁵R⁶; CN ; un radical alkyle en C₁-C₂₀, linéaire ou ramifié ; un radical alcényle en C₂- C₁₀; un radical cycloalkyle en *bicycloalcényle*C₃-C₁₀ ; un radical bicycloalkyle en C₇-C₁₀ ; un radical cycloalcényle en C₃-C₁₀ ; radical bicycloalcényle en C₇-C₁₀ ; un aryle ; un hétéroaryle ;
- R⁵ et R⁶ identiques ou différents, désignent hydrogène; [X]ₚ-R⁷ , C₁-C₆-alkylène-SO₃Y; C₁-C₆-alkylène-PO₃Y ; C₁-C₆-alkylène-N(R⁸)₃⁺ A⁻; un radical alkyle en C₁-C₂₀, linéaire ou ramifié ; un radical alcényle en C₂-C₁₀ ; un radical cycloalkyle en C₃-C₁₀ ; un radical bicycloalkyle en C₇-C₁₀ ; un radical cycloalcényle en C₃-C₁₀ ; un radical bicycloalcényle en C₇-C₁₀ ; un aryle ; un hétéroaryle ;
- X désigne un groupe -CH₂-CH₂-Z-, -CH₂CH₂CH₂Z-, -CH(CH₃)-CH₂-Z-, -CH₂-CH₂₋CH₂-CH₂-Z-, -CH₂-CH(CH₂CH₃)-Z- :
- A désigne Cl, Br, l, SO₄R⁹ ;
- Y désigne hydrogène, Na⁺, K⁺, Mg²⁺, Ca²⁺, Li⁺, Al³⁺, -N(R⁸)₄⁺
- Z désigne O ou NH ;
- R⁷ et R⁸ identiques ou différents, désignent hydrogène, un radical alkyle en C₁-C₆, linéaire ou ramifié , un radical alcényle en C₂- C₆, linéaire ou ramifié ; un radical acyle en C₁-C₆ linéaire ou ramifié ;
- R⁹ désigne hydrogène, un radical alkyle en C₁-C₆, linéaire ou ramifié ; un radical alcényle en C₂- C₆ ;
- n varie de 1 à 3 ;
- p varie de 0 à 150.

5. Composition selon la revendication 4, où le composé de formule (I) est choisi parmi ceux de formule (la) suivante : 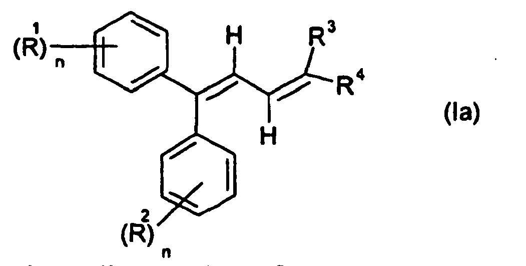 dans laquelle le système diène est de configuration Z,Z ; Z,E ; E,Z ou E,E ou des mélanges desdites configurations et où :
- R¹ et R², identiques ou différents, désignent hydrogène, un radical alkyle en C₁-C₈ ; un radical alcoxy en C₁-C₈ ; un substituant hydrosolubilisant choisi parmi un groupe carboxylate, un groupe sulfonate ou un reste ammonium ;
- R³ désigne un groupe COOR⁵ ; CONR⁵R⁶ ; CN ;
- R⁴ désigne un groupe COOR⁶ ; CONR⁵R⁶ ;
- R⁵ désigne hydrogène ; [X]ₚ-R⁷ ; C₁-C₆-alkylène-SO₃Y ; C₁-C₆-alkylène-N(R⁸)₃⁺ A-;
- R⁶ désigne [X]ₚ-R⁷ ; C₁-C₆-alkylène-SO₃Y ; C₁-C₆-alkylène-N(R⁸)₃⁺ A⁻ ;
- X désigne un groupe -CH₂-CH₂-O-, -CH₂CH₂CH₂O-, -CH(CH₃)-CH₂-O-,
- A désigne Cl, Br, I, SO₄R⁹ ;
- Y désigne hydrogène. Na⁺, K⁺, Mg²⁺, Ca²⁺, Li⁺, Al³⁺, -N(R⁸)₄⁺
- R⁷, R⁸ et R⁹ identiques ou différents, désignent hydrogène, un radical alkyle en C₁-C₃, linéaire ou ramifié ;
- n varie de 1 à 3
- p varie de 0 à 50.

6. Composition selon la revendication **5**, où le composé de formule **(I)** est choisi parmi ceux de formule formule (Ib) suivante : 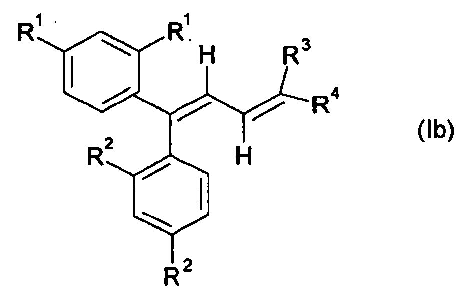 dans laquelle le système diène est de configuration Z,Z ; Z,E ; E,Z ou E,E ou des mélanges desdites configurations et où :
- R¹ et R², identiques ou différents, désignent hydrogène, un radical alkyle en C₁-C₈ ; un radical alcoxy en C₁-C₈;
- R³ désigne un groupe COOR⁵; CONR⁵R⁸ ; CN;
- R⁴ désigne un groupe COOR⁶ ; CONR⁵R⁶ ;
- R⁵ désigne hydrogène : [X]ₚ-R⁷; ;C₁-C₆-alkylène-SO₃Y, C₁-C₆-alkylène-N(R⁸)₃' A⁻;
- R⁶ désigne [X]p-R⁷; C₁-C₆-alkylène-SO₃Y; C₁-C₆-alkylène-N(R⁸)₃⁺A⁻;
- X désigne un groupe -CH₂-CH₂-O-, -CH₂CH₂CH₂O-, -CH(CH₃)-CH₂-O-,
- A désigne CI, Br, l, SO₄R⁹;
- Y désigne hydrogène, Na⁺, K⁺, Mg²⁺, Ca²⁺, Li⁺, Al³⁺, -N(R⁸)₄⁺
- R⁷, R⁸ et R identiques ou différents, désignent hydrogène, un radical alkyle en C₁-C_{3.} linéaire ou ramifié ;
- p varie de 0 à 50.

7. Composition selon la revendication **6**, où le composé de formule (I) est choisi parmi ceux répondant à la formule (Ic) suivante : 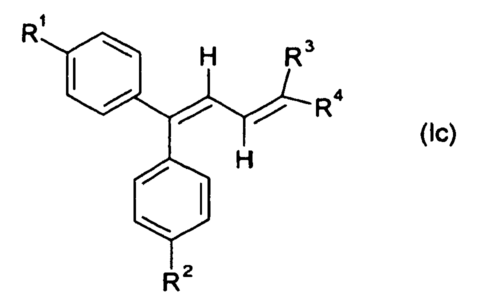 dans laquelle le système diène est de configuration Z,Z ; Z,E ; E,Z ou E,E ou des mélanges desdites configurations et où :
- R¹ et R², identiques ou différents, désignent hydrogène, un radical alkyle en C₁-C₈; un radical alcoxy en C₁-C₈ ;
- R³ désigne un groupe COOR⁵ ; CONR⁵R⁶ ; CN ;
- R⁴ désigne un groupe COOR⁶ ; CONR⁵R⁶ ;
- R⁵ désigne hydrogène ; [X]ₚ-R⁷ ; C₁-C₆-alkylène-SO₃Y; C₁-C₆-alkylène-N(R⁸)₃⁺ A⁻ ;
- R⁶ désigne [X]ₚ-R⁷; C₁-C₆-alkylène-SO₃Y ; C₁-C₆-alkylène-N(R⁸)₃⁺ A⁻ ;
- X désigne un groupe -CH₂-CH₂-O-, -CH₂CH₂CH₂O-, -CH(CH₃)-CH₂-O-,
- A désigne Cl, Br, I, SO₄R⁹ ;
- Y désigne hydrogène, Na⁺, K⁺, Mg²⁺, Ca²⁺, Li⁺, Al³⁺, -N(R⁸)₄⁺
- R⁷, R⁸ et R⁹ identiques ou différents, désignent hydrogène, un radical alkyle en C₁-C₃, linéaire ou ramifié ;
- p varie de 0 à 50.

8. Composition selon la revendication **6 ou 7** où le composé de formule (I) est choisi parmi les composés suivants :

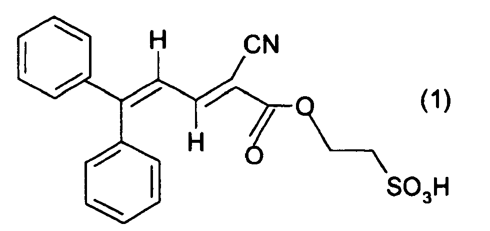

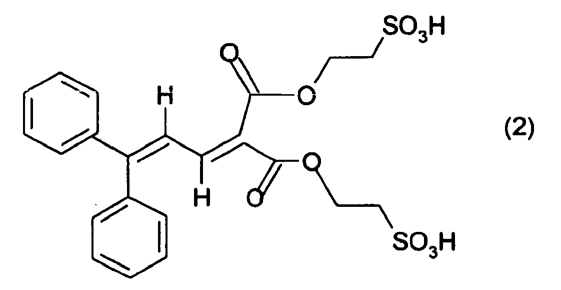

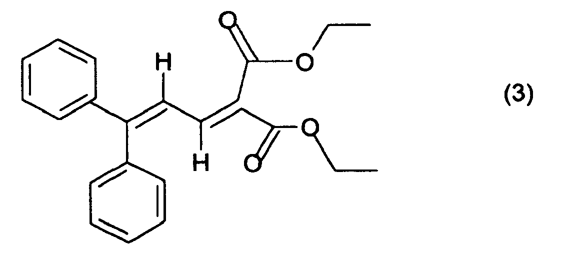

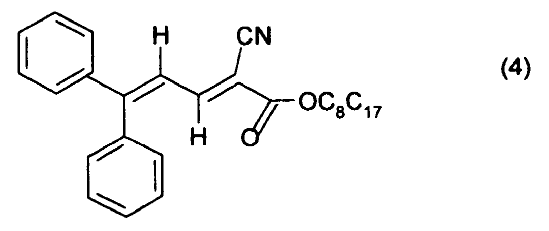

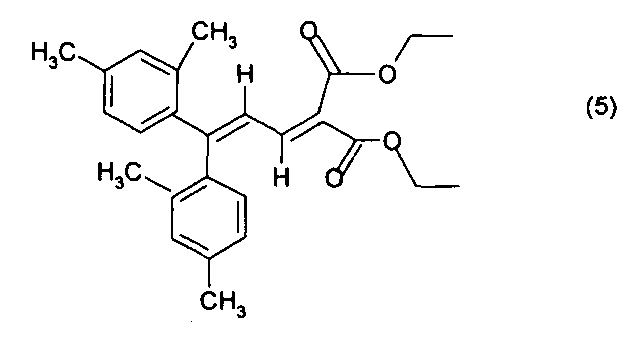

9. Composition **selon l'une quelconque des revendications 1 à 3,** où le composé 4,4-diarylbutadiène est un oligomère répondant à la formule (II) suivante : 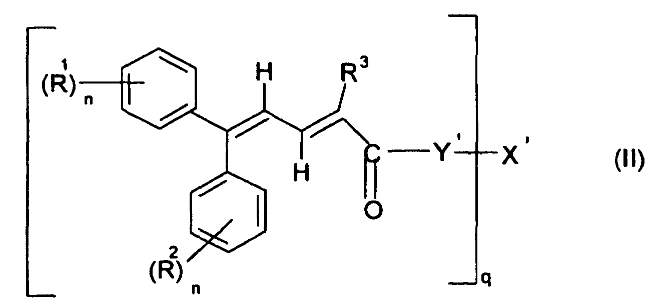 dans laquelle le système diène est de configuration Z,Z ; Z,E ; E,Z ou E,E ou des mélanges desdites configurations et où :
- R¹, R² R³ et n ont les mêmes significations indiquées dans la formule (1) dans la revendication 2 ;
- Y' désigne un groupe -O- ou -NR¹⁰-
- R¹⁰ désigne hydrogène ; un radical alkyle en C₁-C₂₀, linéaire ou ramifié ; un radical alcényle en C₂- C₁₀ ; un radical cycloalkyle en C₃-C₁₀ ; un radical bicycloalkyle en C₇-C₁₀ ; un radical cycloalcényle en C₃-C₁₀ ; ; un radical bicycloalcényle en C₇-C₁₀ ; un aryle ; un hétéroaryle ;
- X' désigne un reste de polyol linéaire ou ramifié, aliphatique ou cycloaliphatique comprenant de 2 à 10 groupes hydroxy et de valence q ; la chaîne carbonée dudit reste pouvant être interrompue par un ou plusieurs atomes de souffre ou d'oxygène ; un ou plusieurs groupes imines ou un ou plusieurs alkylimino en C₁-C₄;
- q varie de 2 à 10.

10. Composition selon la revendication **9,** où le composé de formule (II) est choisi parmi ceux pour lesquels :
- R¹ et R² identiques ou différents, désignent hydrogène, un radical alkyle en C₁-C₁₂; un radical alcoxy en C₁-C₈; un substituant hydrosolubilisant choisi parmi un groupe carboxylate, un groupe sulfonate ou un reste ammonium ;
- R désigne un groupe COOR⁵; CONR⁵R⁶; CN ; un radical cycloalkyle en C₃-C₁₀ ; un radical bicycloalkyle en C₇-C₁₀ ;
- R⁵ et R⁶ , identiques ou différents, désignent un radical alkyle en C₁-C₂₀, linéaire ou ramifié ; un radical cycloalkyle en C₃-C₁₀ ; un radical bicycloalkyle en C₇-C₁₀ ; naphtyle ou phényle éventuellement substitué ;
- X' désigne un reste de polyol comprenant de 2 à 6 groupes hydroxy et plus particulièrement de 2 à 4.

11. Composition selon la revendication **10**, où le composé de formule (II) est choisi parmi ceux pour lesquels :
- X' désigne un reste d'éthanol ou de pentaérythrol.

12. Composition selon la revendication **11,** où le composé de formule (II) est choisi parmi les composés suivants :

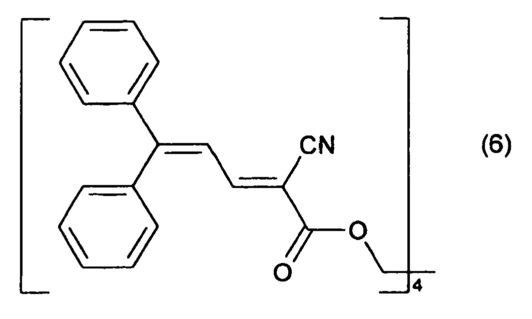

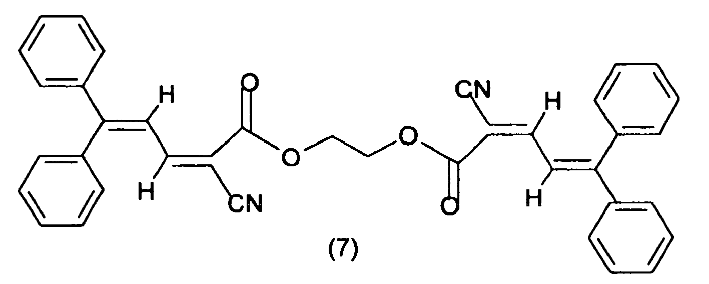

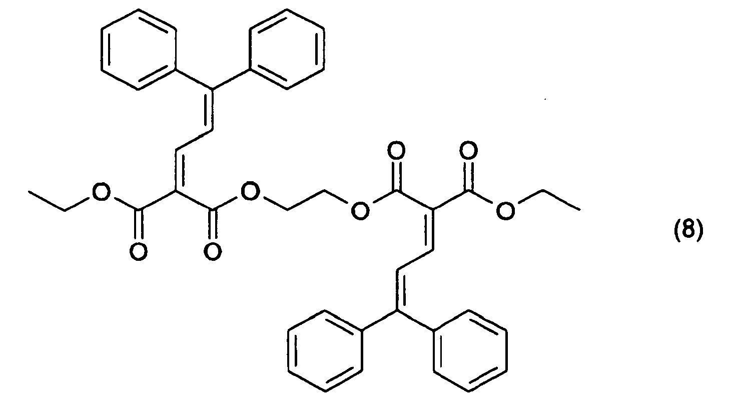

13. Composition selon l'une quelconque des revendications **1** à **12, caractérisée par le fait que** le dérivé de dibenzoylméthane est choisi parmi :
- le 2-méthyldibenzoylméthane
- le 4-méthyldibenzoylméthane
- le 4-isopropyldibenzoylméthane
- le 4-tert.-butyldibenzoylméthane
- le 2,4-diméthyldibenzoylméthane
- le 2,5-diméthyldibenzoylméthane
- le 4,4'-diisopropyldibenzoylméthane
- le 4,4'-diméthoxydibenzoylméthane
- le 4-tert.-butyl-4'-méthoxydibenzoylméthane
- le 2-méthyl-5-isopropyl-4'-méthoxydibenzoylméthane
- le 2-méthyl-5-tert.-butyl-4'-méthoxydibenzoylméthane
- le 2,4-diméthyl-4'-méthoxydibenzoylméthane.
- le 2,6-diméthyl-4-tert.-butyl-4'-méthoxydibenzoylméthane.

14. Composition selon la revendication **13, caractérisée par le fait que** le dérivé de dibenzoylméthane est le 4-(ter.-butyl) 4'-méthoxy dibenzoylméthane.

15. Composition selon la revendication **13, caractérisée par le fait que** le dérivé de dibenzoylméthane est le 4-isopropyl-dibenzoylméthane.

16. Composition selon l'une quelconque des revendications 1 à **15,** où le composé 4,4-diarylbutadiène est présent à des teneurs allant de 0,5 % à 15 % en poids, par rapport au poids total de la composition.

17. Composition selon la revendication **16,** où le composé 4,4-diarylbutadiène est présent à des teneurs allant de 1 % à 10% en poids, par rapport au poids total de la composition.

18. Composition selon l'une quelconque des revendications 1 à **17,** où le dérivé du dibenzoylméthane est présent à des teneurs allant de 0,5 % à 15 % en poids, par rapport au poids total de la composition.

19. Composition selon la revendication **18,** où le dérivé du dibenzoylméthane est présent à des teneurs allant de 1 % à 10% en poids, par rapport au poids total de la composition.

20. Composition selon l'une quelconque des revendications 1 à **19, caractérisée par le fait qu'**elle contient en plus d'autres filtres organiques actifs dans l'uV-A et/ou l'UV-B.

21. Composition selon la revendication **20,** où le ou les filtres UV organiques complémentaires sont choisis parmi les anthranilates; les dérivés salicyliques, les dérivés du camphre ; les dérivés de la benzophénone ; les dérivés de β,β'-diphénylacrylate, les dérivés de benzotriazole ; les dérivés de benzalmalonate ; les dérivés de benzimidazole ; les imidazolines ; les dérivés bis-benzoazolyle les dérivés de l'acide p-aminobenzoïque (PABA) ; les dérivés de méthylène bis-(hydroxyphényl benzotriazole) ; les polymères filtres et silicones filtres ; les dimères dérivés d'α-alkylstyrène.

22. Composition selon la revendication **21, caractérisée par le fait que** le ou les filtres UV organiques sont choisis parmi les composés suivants:
- Ethylhexyl Salicylate,
- Octocrylene,
- Phenylbenzimidazole Sulfonic Acid,
- Terephthalylidene Dicamphor Sulfonic Acid,
- Benzophenone-3.
- Benzophenone-4,
- Benzophenone-5,
- 4-Methylbenzylidene camphor,
- Disodium Phenyl Dibenzimidazole Tetra-sulfonate
- Anisotriazine,
- Ethylhexyl triazone,
- Diethylhexyl Butamido Triazone,
- la 2,4,6-tris-(4'-amino benzalmalonate de diisobutyle)-s- triazine
- Methylene bis-Benzotriazolyl Tetramethylbutylphenol,
- Drometrizole Trisiloxane,
et leurs mélanges.

23. Composition selon l'une quelconque des revendications 1 à **22, caractérisée par le fait qu'**elle comprend en outre, des pigments ou des nanopigments d'oxydes métalliques, enrobés ou non.

24. Composition selon la revendication **23, caractérisée par le fait que** lesdits pigments ou nanopigments sont choisis parmi les oxydes de titane, de zinc, de fer, de zirconium, de cérium et leurs mélanges, enrobés ou non.

25. Composition selon l'une quelconque des revendications 1 à **24, caractérisée par le fait qu'**elle comprend en outre au moins un agent de bronzage et/ou de brunissage artificiel de la peau.

26. Composition selon l'une quelconque des revendications 1 à **25, caractérisée par le fait qu'**elle comprend en outre au moins un adjuvant choisi parmi les corps gras, les solvants organiques, les épaississants ioniques ou non ioniques, les adoucissants, les antioxydants, les agents anti-radicaux libres, les opacifiants, les stabilisants, les émollients, les silicones, les α-hydroxyacides, les agents anti-mousse, les agents hydratants, les vitamines, les agents répulsifs contre les insectes, les parfums, les conservateurs, les tensioactifs, les anti-inflammatoires, les antagonistes de substance P, les charges, les polymères, les propulseurs, les agents alcalinisants ou acidifiants, les colorants.

27. Utilisation d'un composé 4,4-diarylbutadiène tel que défini dans l'une quelconque des revendications précédentes dans une composition cosmétique comprenant au moins un filtre UV du type dérivé du dibenzoylméthane dans le but de d'améliorer la stabilité vis-à-vis des rayons UV dudit dérivé du dibenzoylméthane ; **le rapport en poids du composé 4,4-diarylbutadiène sur le dérivé de dibenzoylméthane étant supérieur à 2,5 et ladite composition ne contenant pas de dérivé de cinnamate.**

## Claims

1. Cosmetic or dermatological composition for topical use, **characterized in that** it comprises, in a cosmetically acceptable vehicle:
(a) at least one UV screening agent of the dibenzoylmethane derivative type and,
(b) at least one 4,4-diarylbutadiene compound; the ratio by weight of the 4,4-diarylbutadiene compound to the dibenzoylmethane derivative being greater than 2.5
and the said composition not comprising cinnamate derivative.

2. Composition according to Claim 1, where the 4,4-diarylbutadiene compound corresponds to the following formula (I): 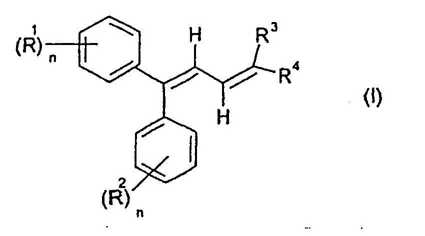 in which the diene system has the Z,Z; Z,E; E,Z or E,E configuration or is composed of mixtures of the said configurations, and where:
- R¹ and R², which are identical or different, denote hydrogen; a linear or branched C₁-C₂₀ alkyl radical; a C₂-C₁₀ alkenyl radical; a C₁-C₁₂ alkoxy radical; a C₃-C₁₀ cycloalkyl radical; a C₃-C₁₀ cycloalkenyl radical; a linear or branched C₁-C₂₀ alkoxycarbonyl radical; a linear or branched C₁-C₁₂ monoalkylamino radical; a linear or branched di (C₁-C₁₂) alkylamino radical; an aryl; a heteroaryl or a water-solubilizing substituent chosen from a carboxylate group, a sulphonate group or an ammonium residue;
- R³ denotes a COOR⁵, COR⁵, CONR⁵R⁶ or CN group; a linear or branched C₁-C₂₀ alkyl radical; a C₂-C₁₀ alkenyl radical; a C₃-C₁₀ cycloalkyl radical; a C₇-C₁₀ bicycloalkyl radical; a C₃-C₁₀ cycloalkenyl radical; a C₇-C₁₀ bicycloalkenyl radical; a C₆-C₁₈ aryl; or a C₃-C₇ heteroaryl;
- R⁴ denotes a COOR⁶, COR⁶, CONR⁵R⁶ or CN group; a linear or branched C₁-C₂₀ alkyl radical; a C₂-C₁₀ alkenyl radical; a C₃-C₁₀ cycloalkyl radical; a C₇-C₁₀ bicycloalkyl radical; a C₃-C₁₀ cycloalkenyl radical; a C₇-C₁₀ bicycloalkenyl radical; an aryl; or a heteroaryl;
- R⁵ and R⁶, which are identical or different, denote hydrogen; [X]ₚ-R⁷; C₁-C₆-alkylene-SO₃Y; C₁-C₆-alkylene-PO₃Y; C₁-C₆-alkylene-N (R⁸) ₃⁺A⁻; a linear or branched C₁-C₂₀ alkyl radical; a C₂-C₁₀ alkenyl radical; a C₃-C₁₀ cycloalkyl radical; a C₇-C₁₀ bicycloalkyl radical; a C₃-C₁₀ cycloalkenyl radical; a C₇-C₁₀ bicycloalkenyl radical; an aryl; or a heteroaryl;
- X denotes a -CH₂-CH₂-Z-, -CH₂CH₂CH₂Z-, -CH (CH₃) - CH₂-Z-, -CH₂-CH₂-CH₂-CH₂-Z- or -CH₂-CH(CH₂CH₃)-Z-group;
- A denotes Cl, Br, I or SO₄R⁹;
- Y denotes hydrogen, Na⁺, K⁺, Mg²⁺, Ca²⁺, Li⁺, Al³⁺ or -N (R⁸)₄⁺;
- Z denotes O or NH;
- R⁷ and R⁸, which are identical or different, denote hydrogen; a linear or branched C₁-C₆ alkyl radical; a linear or branched C₂-C₆ alkenyl radical; or a linear or branched C₁-C₆ acyl radical;
- R⁹ denotes hydrogen; a linear or branched C₁-C₆ alkyl radical; or a C₂-C₆ alkenyl radical;
- n varies from 1 to 3;
- p varies from 0 to 150.

3. Composition according to Claim 2, where the compound of formula (I) is chosen from those of following formula (Ia): 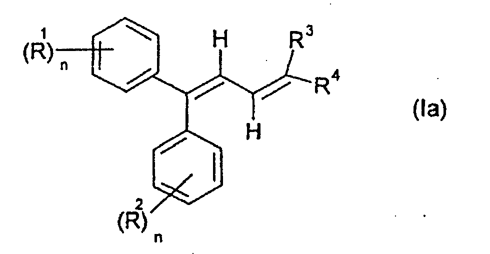 in which the diene system has the Z,Z; Z,E; E,Z or E,E configuration or is composed of mixtures of the said configurations, and where:
- R¹ and R², which are identical or different, denote hydrogen; a C₁-C₈ alkyl radical; a C₁₋C₈ alkoxy radical; or a water-solubilizing substituent chosen from a carboxylate group, a sulphonate group or an ammonium residue;
- R³ denotes a COOR⁵, CONR⁵R⁶ or CN group;
- R⁴ denotes a COOR⁶ or CONR⁵R⁶ group;
- R⁵ denotes hydrogen; [X]ₚ-R⁷; C₁-C₆-alkylene-SO₃Y; or C₁-C₆-alkylene-N(R⁸)₃⁺A⁻;
- R⁶ denotes [X]p-R⁷; C₁-C₆-alkylene-SO₃Y; or C₁-C₆-alkylene-N (R⁸) ₃⁺A⁻;
- X denotes a -CH₂-CH₂-O-, -CH₂CH₂CH₂O- or -CH(CH₃)-CH₂-O- group;
- A denotes Cl, Br, I or SO₄R⁹;
- Y denotes hydrogen, Na⁺, K⁺, Mg²⁺, Ca²⁺, Li⁺, Al³⁺ or -N(R⁸)₄⁺;
- R⁷, R⁸ and R⁹, which are identical or different, denote hydrogen or a linear or branched C₁-C₃ alkyl radical;
- n varies from 1 to 3;
- p varies from 0 to 50.

4. Composition according to Claim 3, where the compound of formula (I) is chosen from those of following formula (Ib): 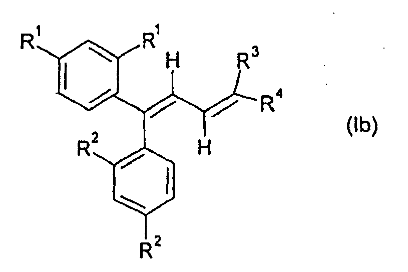 in which the diene system has the Z,Z; Z,E; E,Z or E,E configuration or is composed of mixtures of the said configurations, and where:
- R¹ and R², which are identical or different, denote hydrogen; a C₁-C₈ alkyl radical; or a C₁-C₈ alkoxy radical;
- R³ denotes a COOR⁵, CONR⁵R⁶ or CN group;
- R⁴ denotes a COOR⁶ or CONR⁵R⁶ group;
- R⁵ denotes hydrogen; [X]ₚ-R⁷; C₁-C₆-alkylene-SO₃Y; or C₁-C₆-alkylene-N (R⁸)₃⁺A⁻;
- R⁶ denotes [X]ₚ-R⁷; C₁-C₆-alkylene-SO₃Y; or C₁-C₆-alkylene-N(R⁸)₃⁺A⁻;
- X denotes a -CH₂-CH₂-O-, -CH₂CH₂CH₂O- or -CH(CH₃)-CH₂-O- group;
- A denotes Cl, Br, I or SO₄R⁹;
- Y denotes hydrogen, Na⁺, K⁺, Mg²⁺, Ca²⁺, Li⁺, Al³⁺ or -N(R⁸)₄⁺;
- R⁷, R⁸ and R⁹, which are identical or different, denote hydrogen or a linear or branched C₁-C₃ alkyl radical;
- p varies from 0 to 50.

5. Composition according to Claim 4, where the compound of formula (I) is chosen from those corresponding to the following formula (Ic): 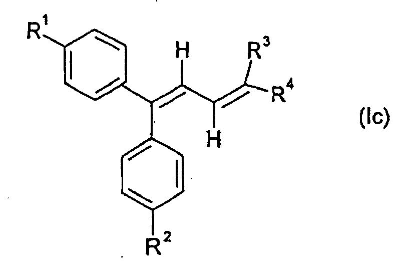 in which the diene system has the Z,Z; Z,E; E,Z or E,E configuration or is composed of mixtures of the said configurations, and where:
- R¹ and R², which are identical or different, denote hydrogen; a C₁-C₈ alkyl radical; or a C₁-C₈ alkoxy radical;
- R³ denotes a COOR⁵, CONR⁵R⁶ or CN group;
- R⁴ denotes a COOR⁶ or CONR⁵R⁶ group;
- R⁵ denotes hydrogen; [X]p-R⁷; C₁-C₆-alkylene-SO₃Y; or C₁-C₆-alkylene-N (R⁸)₃⁺A-;
- R⁶ denotes [X]p-R⁷; C₁-C₆-alkylene-SO₃Y; or C₁-C₆-alkylene-N (R⁸) ₃⁺A-;
- X denotes a -CH₂-CH₂-O-, -CH₂CH₂CH₂O- or -CH(CH₃)-CH₂-O- group;
- A denotes Cl, Br, I or SO₄R⁹;
- Y denotes hydrogen, Na⁺, K⁺, Mg²⁺, Ca²⁺, Li⁺, Al³⁺ or -N(R⁸)₄⁺;
- R⁷, R⁸ and R⁹, which are identical or different, denote hydrogen or a linear or branched C₁-C₃ alkyl radical;
- p varies from 0 to 50.

6. Composition according to Claim 4 or 5, where the compound of formula (I) is chosen from the following compounds:

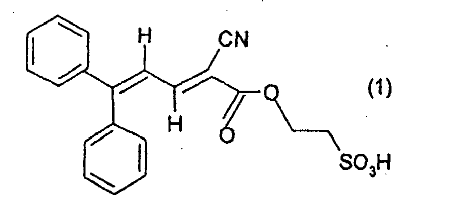

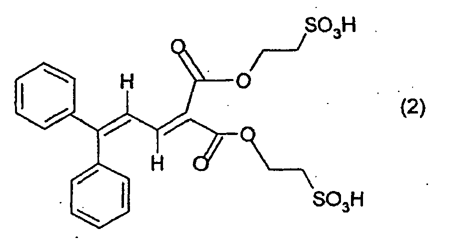

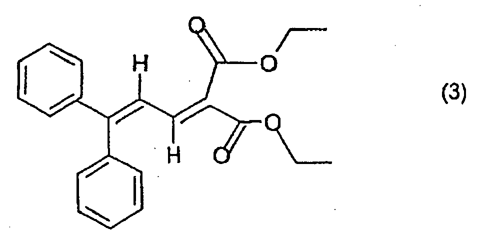

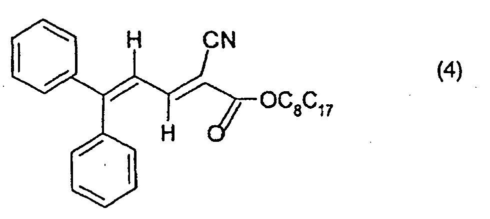

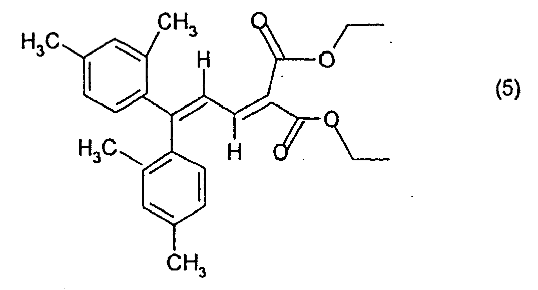

7. Composition according to Claim 1, where the 4,4-diarylbutadiene compound is an oligomer corresponding to the following formula (II): 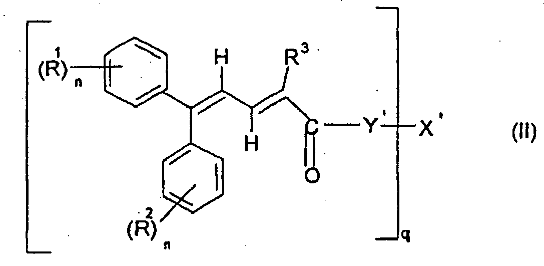 in which the diene system has the Z,Z; Z,E; E,Z or E,E configuration or is composed of mixtures of the said configurations, and where:
- R¹, R², R³ and n have the same meanings indicated in the formula (I) in Claim 2;
- Y' denotes an -O- or -NR¹⁰- group;
- R¹⁰ denotes hydrogen; a linear or branched C₁-C₂₀ alkyl radical; a C₂-C₁₀ alkenyl radical; a C₃-C₁₀ cycloalkyl radical; a C₇-C₁₀ bicycloalkyl radical; a C₃-C₁₀ cycloalkenyl radical; a C₇-C₁₀ bicycloalkenyl radical; an aryl; or a heteroaryl;
- X' denotes a linear or branched, aliphatic or cycloaliphatic, polyol residue comprising 2 to 10 hydroxyl groups and with a valency of q; it being possible for the carbonaceous chain of the said residue to be interrupted by one or more sulphur or oxygen atoms; one or more imine groups; or one or more C₁-C₄ alkylimine groups;
- q varies from 2 to 10.

8. Composition according to Claim 7, where the compound of formula (II) is chosen from those for which:
- R¹ and R ² which are identical or different, denote hydrogen; a C₁-C₁₂ alkyl radical; a C₁-C₈ alkoxy radical; or a water-solubilizing substituent chosen from a carboxylate group, a sulphonate group or an ammonium residue;
- R³ denotes a COOR⁵, CONR⁵R⁶or CN group; a C₃-C₁₀ cycloalkyl radical; or a C₇-C₁₀ bicycloalkyl radical;
- R⁵ and R⁶, which are identical or different, denote a linear or branched C₁-C₂₀ alkyl radical; a C₃-C₁₀ cycloalkyl radical; a C₇-C₁₀ bicycloalkyl radical; or an optionally substituted naphthyl or phenyl;
- X' denotes a polyol residue comprising from 2 to 6 hydroxyl groups and more particularly from 2 to 4.

9. Composition according to Claim 8, where the compound of formula (II) is chosen from those for which:
- X' denotes an ethanol or pentaerythritol residue.

10. Composition according to Claim 9, where the compound of formula (II) is chosen from the following compounds:

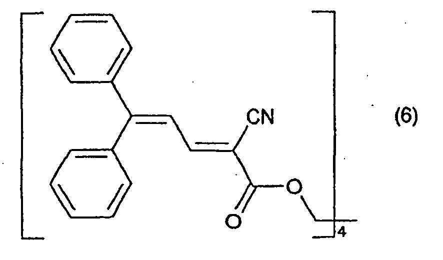

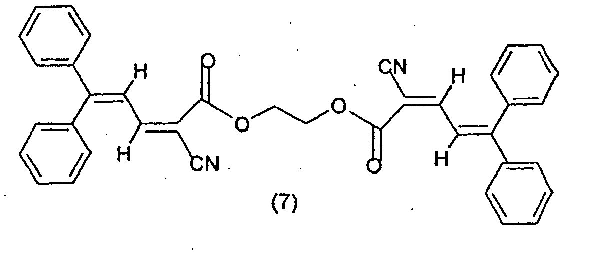

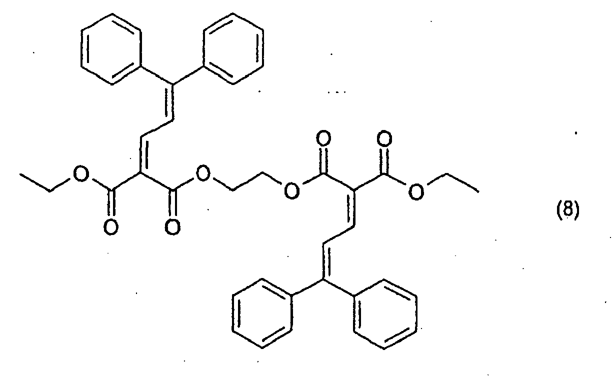

11. Composition according to any one of Claims 1 to 10, **characterized in that** the dibenzoylmethane derivative is chosen from:
- 2-methyldibenzoylmethane,
- 4-methyldibenzoylmethane,
- 4-isopropyldibenzoylmethane,
- 4-tert-butyldibenzoylmethane,
- 2,4-dimethyldibenzoylmethane,
- 2,5-dimethyldibenzoylmethane,
- 4,4'-diisopropyldibenzoylmethane,
- 4,4'-dimethoxydibenzoylmethane,
- 4-tert-butyl-4'-methoxydibenzoylmethane,
- 2-methyl-5-isopropyl-4'-methoxydibenzoylmethane,
- 2-methyl-5-tert-butyl-4'-methoxydibenzoylmethane,
- 2,4-dimethyl-4'-methoxydibenzoylmethane,
- 2,6-dimethyl-4-tert-butyl-4'-methoxydibenzoylmethane.

12. Composition according to Claim 11, **characterized in that** the dibenzoylmethane derivative is 4-(tert-butyl)-4'-methoxydibenzoylmethane.

13. Composition according to Claim 12, **characterized in that** the dibenzoylmethane derivative is 4-isopropyldibenzoylmethane.

14. Composition according to any one of Claims 1 to 13, where the 4,4'-diarylbutadiene compound is present at contents ranging from 0.5% to 15% by weight, with respect to the total weight of the composition.

15. Composition according to Claim 14, where the 4,4-diarylbutadiene compound is present at contents ranging from 1% to 10% by weight, with respect to the total weight of the composition.

16. Composition according to any one of Claims 1 to 14, where the dibenzoylmethane derivative is present at contents ranging from 0.5% to 15% by weight, with respect to the total weight of the composition.

17. Composition according to Claim 16, where the dibenzoylmethane derivative is present at contents ranging from 1% to 10% by weight, with respect to the total weight of the composition.

18. Composition according to any one of Claims 1 to 17, **characterized in that** it additionally comprises other organic screening agents which are active in the UV-A and/or UV-B regions.

19. Composition according to Claim 16, where the additional organic UV screening agent or agents are chosen from anthranilates; salicylic derivatives; camphor derivatives; benzophenone derivatives; β,β-diphenylacrylate derivatives; benzotriazole derivatives; benzalmalonate derivatives; benzimidazole derivatives; imidazolines; bisbenzoazolyl derivatives; p-aminobenzoic acid (PABA) derivatives; methylenebis(hydroxyphenylbenzotriazole) derivatives; screening polymers and screening silicones; or dimers derived from α-alkylstyrene.

20. Composition according to Claim 19, **characterized in that** the organic UV screening agent or agents are chosen from the following compounds:
- Ethylhexyl Salicylate,
- Octocrylene,
- Phenylbenzimidazole Sulfonic Acid,
- Terephthalylidene Dicamphor Sulfonic Acid,
- Benzophenone-3,
- Benzophenone-4,
- Benzophenone-5,
- 4-Methylbenzylidene camphor,
- Disodium Phenyl Dibenzimidazole Tetrasulfonate,
- Anisotriazine,
- Ethylhexyl Triazone,
- Diethylhexyl Butamido Triazone,
- 2,4,6-Tris(diisobutyl 4'-aminobenzalmalonate)-s-triazine,
- Methylene bis-Benzotriazolyl Tetramethylbutylphenol,
- Drometrizole Trisiloxane,
and their mixtures.

21. Composition according to any one of Claims 1 to 20, **characterized in that** it additionally comprises coated or uncoated metal oxide pigments or nanopigments.

22. Composition according to Claim 19, **characterized in that** the said pigments or nanopigments are chosen from coated or uncoated titanium oxide, zinc oxide, iron oxide, zirconium oxide or cerium oxide, and their mixtures.

23. Composition according to any one of Claims 1 to 22, **characterized in that** it additionally comprises at least one agent for the artificial tanning and/or browning of the skin.

24. Composition according to any one of Claims 1 to 23, **characterized in that** it additionally comprises at least one adjuvant chosen from fatty substances, organic solvents, ionic or nonionic thickeners, softeners, antioxidants, agents for combating free radicals, opacifiers, stabilizers, emollients, silicones, α-hydroxy acids, antifoaming agents, moisturizing agents, vitamins, insect repellents, fragrances, preservatives, surfactants, anti-inflammatories, substance P antagonists, fillers, polymers, propellants, basifying or acidifying agents, or colorants.

25. Composition according to any one of Claims 1 to 24, **characterized in that** it is a protective composition for the human epidermis or an antisun composition and **in that** it is provided in the form of a nonionic vesicular dispersion, of an emulsion, in particular of an emulsion of oil-in-water type, of a cream, of a milk, of a gel, of a cream gel, of a suspension, of a dispersion, of a powder, of a solid tube, of a foam or of a spray.

26. Composition according to any one of Claims 1 to 24, **characterized in that** it is a composition for making up the eyelashes, eyebrows or skin and **in that** it is provided in the anhydrous or aqueous, pasty or solid form, in the form of an emulsion, of a suspension or of a dispersion.

27. Composition according to any one of Claims 1 to 24, **characterized in that** it is a composition intended for the protection of the hair against ultraviolet rays and **in that** it is provided in the form of a shampoo, of a lotion, of a gel, of an emulsion or of a nonionic vesicular dispersion.

28. Use of a 4,4-diarylbutadiene compound as defined in any one of the preceding claims in a cosmetic composition comprising at least one UV screening agent of the dibenzoylmethane derivative type for the purpose of improving the stability with regard to UV rays of the said dibenzoylmethane derivative; the ratio by weight of the 4,4-diarylbutadiene compound to the dibenzoylmethane derivative being greater than 2.5 and the said composition not comprising cinnamate derivative.

## Claims (Claims for the following Contracting State(s): DE)

1. Cosmetic or dermatological composition for topical use for the protection of the human epidermis against UV rays, **characterized in that** it comprises, in a cosmetically acceptable vehicle:
(a) at least one UV screening agent of the dibenzoylmethane derivative type and
(b) at least one 4,4-diarylbutadiene compound, the ratio by weight of the 4,4-diarylbutadiene compound to the dibenzoylmethane derivative being greater than 2.5 and
the said composition not comprising cinnamate derivative and **in that** it is provided in the form of a nonionic vesicular dispersion; in the form of an oil/water emulsion; in the form of a gel; in the form of a powder; in the form of a solid stick; or in the form of an aerosol foam or of a spray.

2. Cosmetic or dermatological composition for topical use for the protection of the hair against UV rays, **characterized in that** it comprises, in a cosmetically acceptable vehicle:
(a) at least one UV screening agent of the dibenzoylmethane derivative type and
(b) at least one 4,4-diarylbutadiene compound; the ratio by weight of the 4,4-diarylbutadiene compound to the dibenzoylmethane derivative being greater than 2.5 and
the said composition not comprising cinnamate derivative and **in that** it is provided in the form of a shampoo, of a gel or of a nonionic vesicular dispersion.

3. Composition for making up the eyelashes, eyebrows or skin, **characterized in that** it comprises, in a cosmetically acceptable vehicle:
(a) at least one UV screening agent of the dibenzoylmethane derivative type and
(b) at least one 4,4-diarylbutadiene compound; the ratio by weight of the 4,4-diarylbutadiene compound to the dibenzoylmethane derivative being greater than 2.5 and
the said composition not comprising cinnamate derivative and **in that** it is provided in the anhydrous or aqueous, pasty or solid form, in the form of an oil/water emulsion or in the form of a nonionic vesicular dispersion.

4. Composition according to any one of Claims 1 to 3, where the 4,4-diarylbutadiene compound corresponds to the following formula (I): 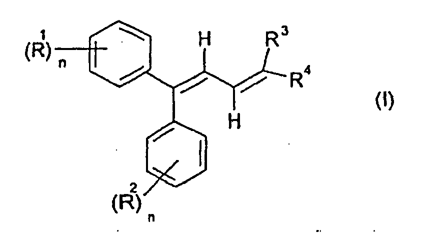 in which the diene system has the Z , Z; Z, E; E, Z or E,E configuration or is composed of mixtures of the said configurations, and where:
- R¹ and R², which are identical or different, denote hydrogen; a linear or branched C₁-C₂₀ alkyl radical; a C₂-C₁₀ alkenyl radical; a C₁-C₁₂ alkoxy radical; a C₃-C₁₀ cycloalkyl radical; a C₃-C₁₀ cycloalkenyl radical; a linear or branched C₁-C₂₀ alkoxycarbonyl radical; a linear or branched C₁-C₁₂ monoalkylamino radical; a linear or branched di (C₁-C₁₂) alkylamino radical; an aryl; a heteroaryl or a water-solubilizing substituent chosen from a carboxylate group, a sulphonate group or an ammonium residue;
- R³ denotes a COOR⁵, COR⁵, CONR⁵R⁶ or CN group; a linear or branched C₁-C₂₀ alkyl radical; a C₂-C₁₀ alkenyl radical; a C₃-C₁₀ cycloalkyl radical; a C₇-C₁₀ bicycloalkyl radical; a C₃-C₁₀ cycloalkenyl radical; a C₇-C₁₀ bicycloalkenyl radical; a . C₆-C₁₈ aryl; or a C₃-C₇ heteroaryl;
- R⁴ denotes a COOR⁶ COR⁶, CONR⁵R⁶ or CN group; a linear or branched C₁-C₂₀ alkyl radical; a C₂-C₁₀ alkenyl radical; a C₃-C₁₀ cycloalkyl radical; a C₇-C₁₀ bicycloalkyl radical; a C₃-C₁₀ cycloalkenyl radical; a C₇-C₁₀ bicycloalkenyl radical; an aryl; or a heteroaryl;
- R⁵ and R⁶, which are identical or different, denote hydrogen; [X]ₚ-R⁷; C₁-C₆-alkylene-SO₃Y; C₁-C₆-alkylene-PO₃Y; C₁-C₆-alkylene-N(R⁸)₃⁺A⁻; a linear or branched C₁-C₂₀ alkyl radical; a C₂-C₁₀ alkenyl radical; a C₃-C₁₀ cycloalkyl radical; a C₇-C₁₀ bicycloalkyl radical; a C₃-C₁₀ cycloalkenyl radical; a C₇-C₁₀ bicycloalkenyl radical; an aryl; or a heteroaryl;
- X denotes a -CH₂-CH₂-Z-, -CH₂CH₂CH₂Z-, -CH (CH₃)-CH₂-Z-, -CH₂-CH₂-CH₂-CH₂-Z- or -CH₂-CH (CH₂CH₃) -Z-group;
- A denotes Cl, Br, I or SO₄R⁹;
- Y denotes hydrogen, Na⁺, K⁺, Mg²⁺, Ca²⁺, Li⁺, Al³⁺ or -N(R⁸)₄⁺;
- Z denotes O or NH;
- R⁷ and R⁸, which are identical or different, denote hydrogen; a linear or branched C₁-C₆ alkyl radical; a linear or branched C₂-C₆ alkenyl radical; or a linear or branched C₁-C₆ acyl radical;
- R⁹ denotes hydrogen; a linear or branched C₁-C₆ alkyl radical; or a C₂-C₆ alkenyl radical;
- n varies from 1 to 3;
- p varies from 0 to 150.

5. Composition according to Claim 4, where the compound of formula (I) is chosen from those of following formula (Ia): 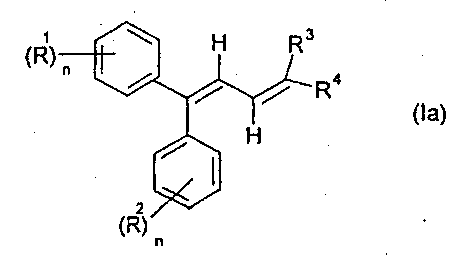 in which the diene system has the Z,Z; Z, E; E, Z or E,E configuration or is composed of mixtures of the said configurations, and where:
- R¹ and R², which are identical or different, denote hydrogen; a C₁-C₈ alkyl radical; a C₁-C₈ alkoxy radical; or a water-solubilizing substituent chosen from a carboxylate group, a sulphonate group or an ammonium residue;
- R³ denotes a COOR⁵, CONR⁵R⁶ or CN group;
- R⁴ denotes a COOR⁶ or CONR⁵R⁶ group;
- R⁵ denotes hydrogen; [X]ₚ-R⁷; C₁-C₆-alkylene-SO₃Y; or C₁-C₆-alkylene-N (R⁸) ₃⁺A⁻;
- R⁶ denotes [X]ₚ-R⁷; C₁-C₆-alkylene-SO₃Y; or C₁-C₆-alkylene-N (R⁸)₃⁺A⁻;
- X denotes a -CH₂-CH₂-O-, -CH₂CH₂CH₂O- or -CH(CH₃)-CH₂-O- group;
- A denotes Cl, Br, I or SO₄R⁹;
- Y denotes hydrogen, Na⁺, K⁺, Mg ²⁺, Ca²⁺, Li⁺, Al³⁺ or -N(R⁸)₄⁺ ;
- R⁷, R⁸ and R⁹, which are identical or different, denote hydrogen or a linear or branched C₁-C₃ alkyl radical;
- n varies from 1 to 3;
- p varies from 0 to 50.

6. Composition according to Claim 5, where the compound of formula (I) is chosen from those of following formula (Ib): 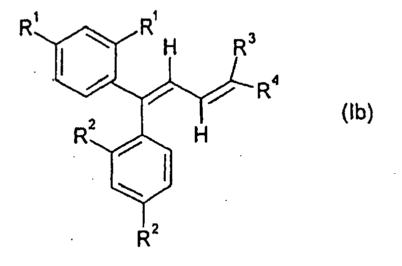 in which the diene system has the Z,Z; Z,E; E,Z or E,E configuration or is composed of mixtures of the said configurations, and where:
- R¹ and R² which are identical or different, denote hydrogen; a C₁-C₈ alkyl radical; or a C₁-C₈ alkoxy radical;
- R³ denotes a COOR⁵, CONR⁵R⁶ or CN group;
- R⁴ denotes a COOR⁶ or CONR⁵R⁶ group;
- R⁵ denotes hydrogen; [X]ₚ-R⁷; C₁-C₆-alkylene-SO₃Y; or C₁-C₆-alkylene-N(R⁸)₃⁺A⁻;
- R⁶ denotes [X]ₚ-R⁷; C₁-C₆-alkylene-SO₃Y; or C₁-C₆-alkylene-N (R⁸)₃⁺A⁻;
- X denotes a -CH₂-CH₂-O-, -CH₂CH₂CH₂O- or -CH(CH₃)-CH₂-O- group;
- A denotes Cl, Br, I or SO₄R⁹;
- Y denotes hydrogen, Na⁺, K⁺, Mg²⁺, Ca²⁺, Li⁺, Al³⁺ or -N (R⁸)₄⁺_{;}
- R⁷, R⁸ and R⁹, which are identical or different, denote hydrogen or a linear or branched C₁-C₃ alkyl radical;
- p varies from 0 to 50.

7. Composition according to Claim 6, where the compound of formula (I) is chosen from those corresponding to the following formula (Ic): 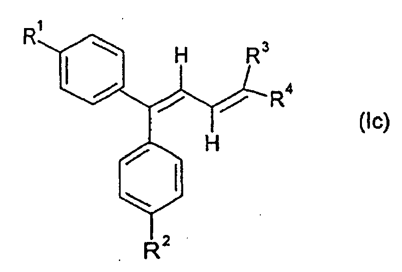 in which the diene system has the Z, Z; Z,E; E,Z or E,E configuration or is composed of mixtures of the said configurations, and where:
- R¹ and R², which are identical or different, denote hydrogen; a C₁-C₈ alkyl radical ; or a C₁-C₈ alkoxy radical;
- R³ denotes a COOR⁵, CONR⁵R⁶ or CN group;
- R⁴ denotes a COOR⁶ or CONR⁵R⁶ group;
- R⁵ denotes hydrogen; [X]ₚ-R⁷; C₁-C₆-alkylene-SO₃Y; or C₁-C₆-alkylene-N(R⁸)₃⁺A⁻;
- R⁶ denotes [X]ₚ-R⁷; C₁-C₆-alkylene-SO₃Y; or C₁-C₆-alkylene-N(R⁸)₃⁺A⁻;
- X denotes a -CH₂-CH₂-O-, -CH₂CH₂CH₂O- or -CH (CH₃)-CH₂-O- group;
- A denotes Cl, Br, I or SO₄R⁹_{;}
- Y denotes hydrogen, Na⁺, K⁺, Mg²⁺, Ca²⁺, Li⁺, Al³⁺ or -N (R⁸) ₄⁺_{;}
- R⁷, R⁸ and R⁹, which are identical or different, denote hydrogen or a linear or branched C₁-C₃ alkyl radical;
- p varies from 0 to 50.

8. Composition according to Claim 6 or 7, where the compound of formula (I) is chosen from the following compounds:

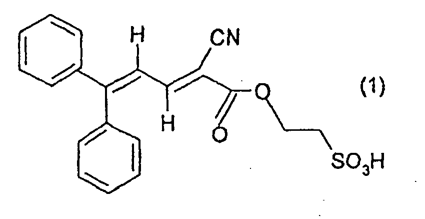

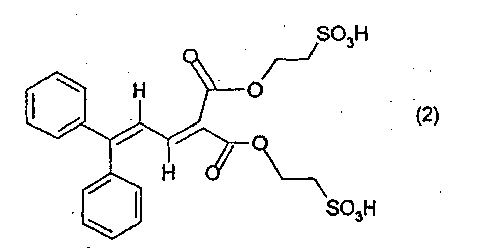

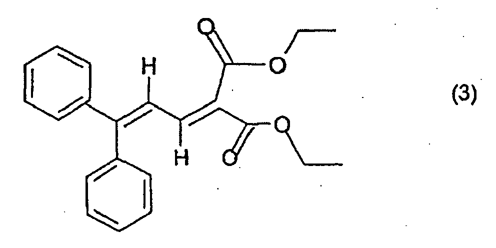

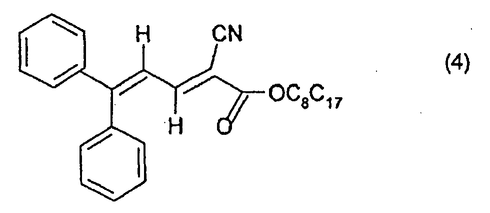

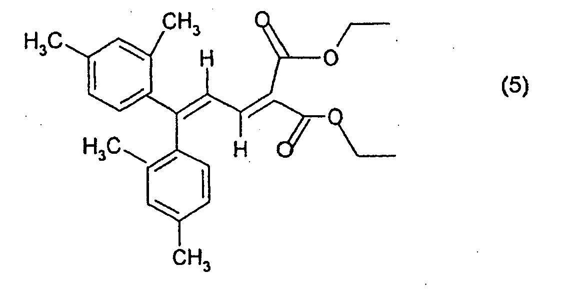

9. Composition according to any one of Claims 1 to 3, where the 4,4-diarylbutadiene compound is an oligomer corresponding to the following formula (II) : 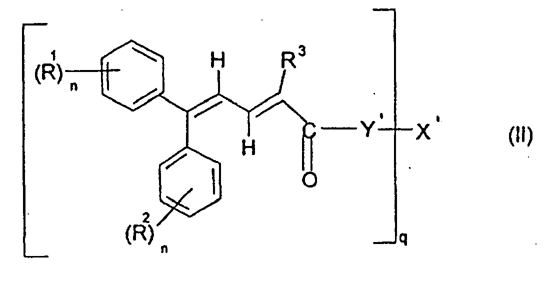 in which the diene system has the Z, Z; Z, E; E,Z or E,E configuration or is composed of mixtures of the said configurations, and where:
- R¹, R², R³ and n have the same meanings indicated in the formula (I) in Claim 2;
- Y' denotes an -O- or -NR¹⁰- group;
- R¹⁰ denotes hydrogen; a linear or branched C₁-C₂₀ alkyl radical; a C₂-C₁₀ alkenyl radical; a C₃-C₁₀ cycloalkyl radical; a C₇-C₁₀ bicycloalkyl radical; a C₃-C₁₀ cycloalkenyl radical; a C₇-C₁₀ bicycloalkenyl radical; an aryl; or a heteroaryl;
- X' denotes a linear or branched, aliphatic or cycloaliphatic, polyol residue comprising 2 to 10 hydroxyl groups and with a valency of q; it being possible for the carbonaceous chain of the said residue to be interrupted by one or more sulphur or oxygen atoms; one or more imine groups; or one or more C₁-C₄ alkylimine groups;
- q varies from 2 to 10.

10. Composition according to Claim 9, where the compound of formula (II) is chosen from those for which:
- R¹ and R², which are identical or different, denote hydrogen; a C₁-C₁₂ alkyl radical; a C₁-C₈ alkoxy radical; or a water-solubilizing substituent chosen from a carboxylate group, a sulphonate group or an ammonium residue;
- R³ denotes a COOR⁵, CONR⁵R⁶ or CN group; a C₃-C₁₀ cycloalkyl radical; or a C₇-C₁₀ bicycloalkyl radical;
- R⁵ and R⁶ which are identical or different, denote a linear or branched C₁-C₂₀ alkyl radical; a C₃-C₁₀ cycloalkyl radical; a C₇-C₁₀ bicycloalkyl radical; or an optionally substituted naphthyl or phenyl;
- X' denotes a polyol residue comprising from 2 to 6 hydroxyl groups and more particularly from 2 to 4.

11. Composition according to Claim 10, where the compound of formula (II) is chosen from those for which:
- X' denotes an ethanol or pentaerythritol residue.

12. Composition according to Claim 11, where the compound of formula (II) is chosen from the following compounds:

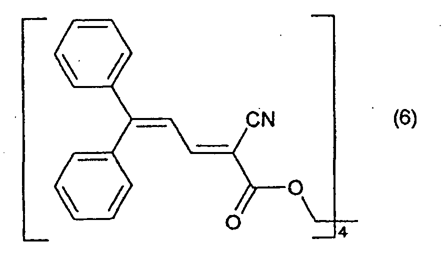

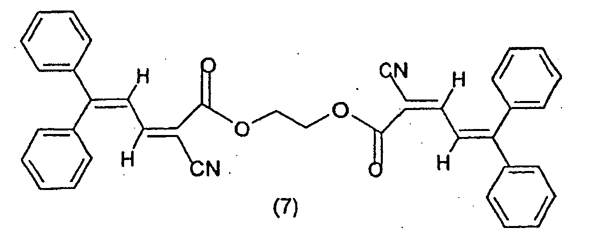

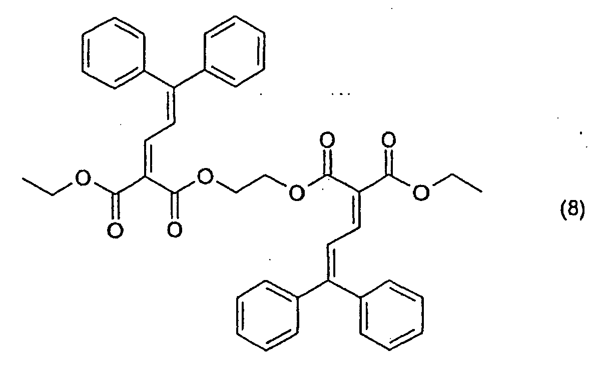

13. Composition according to any one of Claims 1 to 12, **characterized in that** the dibenzoylmethane derivative is chosen from:
- 2-methyldibenzoylmethane,
- 4-methyldibenzoylmethane,
- 4-isopropyldibenzoylmethane,
- 4-tert-butyldibenzoylmethane,
- 2,4-dimethyldibenzoylmethane,
- 2,5-dimethyldibenzoylmethane,
- 4,4'-diisopropyldibenzoylmethane,
- 4,4'-dimethoxydibenzoylmethane,
- 4-tert-butyl-4'-methoxydibenzoylmethane,
- 2-methyl-5-isopropyl-4'-methoxydibenzoylmethane,
- 2-methyl-5-tert-butyl-4'-methoxydibenzoylmethane,
- 2,4-dimethyl-4'-methoxydibenzoylmethane,
- 2,6-dimethyl-4-tert-butyl-4'-methoxydibenzoylmethane.

14. Composition according to Claim 13, **characterized in that** the dibenzoylmethane derivative is 4-(tert-butyl)-4'-methoxydibenzoylmethane.

15. Composition according to Claim 13, **characterized in that** the dibenzoylmethane derivative is 4-isopropyldibenzoylmethane.

16. Composition according to any one of Claims 1 to 15, **characterized in that** the 4,4'-diarylbutadiene compound is present at contents ranging from 0.5% to 15% by weight, with respect to the total weight of the composition.

17. Composition according to Claim 16, where the 4,4-diarylbutadiene compound is present at contents ranging from 1% to 10% by weight, with respect to the total weight of the composition.

18. Composition according to any one of Claims 1 to 17, where the dibenzoylmethane derivative is present at contents ranging from 0.5% to 15% by weight, with respect to the total weight of the composition.

19. Composition according to Claim 18, where the dibenzoylmethane derivative is present at contents ranging from 1% to 10% by weight, with respect to the total weight of the composition.

20. Composition according to any one of Claims 1 to 19, **characterized in that** it additionally comprises other organic screening agents which are active in the UV-A and/or UV-B regions.

21. Composition according to Claim 20, where the additional organic UV screening agent or agents are chosen from anthranilates; salicylic derivatives; camphor derivatives; benzophenone derivatives; β,β-diphenylacrylate derivatives; benzotriazole derivatives; benzalmalonate derivatives; benzimidazole derivatives; imidazolines; bisbenzoazolyl derivatives; p-aminobenzoic acid (PABA) derivatives; methylenebis(hydroxyphenylbenzotriazole) derivatives; screening polymers and screening silicones; or dimers derived from α-alkylstyrene.

22. Composition according to Claim 21, **characterized in that** the organic UV screening agent or agents are chosen from the following compounds:
- Ethylhexyl Salicylate,
- Octocrylene,
- Phenylbenzimidazole Sulfonic Acid,
- Terephthalylidene Dicamphor Sulfonic Acid,
- Benzophenone-3,
- Benzophenone-4,
- Benzophenone-5,
- 4-Methylbenzylidene camphor,
- Disodium Phenyl Dibenzimidazole Tetrasulfonate,
- Anisotriazine,
- Ethylhexyl Triazone,
- Diethylhexyl Butamido Triazone,
- 2,4,6-Tris(diisobutyl 4'-aminobenzalmalonate)-s-triazine,
- Methylene bis-Benzotriazolyl Tetramethylbutylphenol,
- Drometrizole Trisiloxane,
and their mixtures.

23. Composition according to any one of Claims 1 to 22, **characterized in that** it additionally comprises coated or uncoated metal oxide pigments or nanopigments.

24. Composition according to Claim 23, **characterized in that** the said pigments or nanopigments are chosen from coated or uncoated titanium oxide, zinc oxide, iron oxide, zirconium oxide or cerium oxide, and their mixtures.

25. Composition according to any one of Claims 1 to 24, **characterized in that** it additionally comprises at least one agent for the artificial tanning and/or browning of the skin.

26. Composition according to any one of Claims 1 to 25, **characterized in that** it additionally comprises at least one adjuvant chosen from fatty substances, organic solvents, ionic or nonionic thickeners, softeners, antioxidants, agents for combating free radicals, opacifiers, stabilizers, emollients, silicones, α-hydroxy acids, antifoaming agents, moisturizing agents, vitamins, insect repellents, fragrances, preservatives, surfactants, anti-inflammatories, substance P antagonists, fillers, polymers, propellants, basifying or acidifying agents, or colorants.

27. Use of a 4,4-diarylbutadiene compound as defined in any one of the preceding claims in a cosmetic composition comprising at least one UV screening agent of the dibenzoylmethane derivative type for the purpose of improving the stability with regard to UV rays of the said dibenzoylmethane derivative; the ratio by weight of the 4,4-diarylbutadiene compound to the dibenzoylmethane derivative being greater than 2.5 and the said composition not comprising cinnamate derivative.

## Patentansprüche

1. Kosmetische oder dermatologische Zusammensetzung zur topischen Anwendung, **dadurch gekennzeichnet, dass** sie in einem kosmetisch akzeptablen Träger enthält:
(a) mindestens ein UV-Filter vom Typ eines Dibenzoylmethan-Derivats und
(b) mindestens eine 4,4-Diarylbutadien-Verbindung,
wobei das Gewichtsverhältnis von 4,4-Diarylbutadien-Verbindung zu Dibenzoylmethan-Derivat größer als 2,5 ist und die Zusammensetzung kein Cinnamat-Derivat enthält.

2. Zusammensetzung nach Anspruche 1, in der die 4,4-Diarylbutadien-Verbindung die nachstehende Formel (I) aufweist: 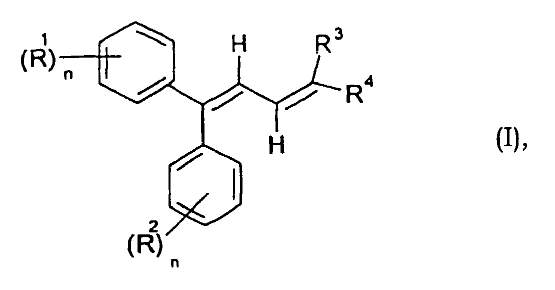 in der das Diensystem die Konfiguration Z,Z; Z,E; E,Z oder E,E aufweist oder Gemische dieser Konfigurationen vorliegen und wobei bedeuten:
- R¹ und R², die gleich oder verschieden sind, Wasserstoff; eine geradkettige oder verzweigte C₁₋₂₀-Alkylgruppe; eine C₂₋₁₀-Alkenylgruppe; eine C₁₋₁₂-Alkoxygruppe; eine C₃₋₁₀-Cycloalkylgruppe; eine C₃₋₁₀-Cycloalkenylgruppe; eine geradkettige oder verzweigte C₁₋₂₀-Alkoxycarbonylgruppe; eine geradkettige oder verzweigte C₁₋₁₂-Monoalkylaminogruppe; eine geradkettige oder verzweigte C₁₋₁₂-Dialkylamiogruppe; eine Arylgruppe, eine Heteroarylgruppe oder einen wasserlöslich machenden Substituenten, der unter einer Carboxylatgruppe, einer Sulfonatgruppe und einem Ammoniumrest ausgewählt ist;
- R³ eine Gruppe COOR⁵; COR⁵; CONR⁵R⁶; CN; eine geradkettige oder verzweigte C₁₋₂₀-Alkylgruppe; eine C₂₋₁₀-Alkenylgruppe, eine C₃₋₁₀-Cycloalkylgruppe; eine C₇₋₁₀-Bicycloalkylgruppe, eine C₃₋₁₀-Cycloalkenylgruppe, eine C₇₋₁₀-Bicycloalkenylgruppe; eine C₆₋₁₈-Arylgruppe; eine C₃₋₇-Heteroarylgruppe;
- R⁴ eine Gruppe COOR⁶; COR⁶; CONR⁵R⁶; CN; eine geradkettige oder verzweigte C₁₋₂₀-Alkylgruppe, eine C₂₋₁₀-Alkenylgruppe; eine C₃₋₁₀-Cycloalkylgruppe; eine C₇₋₁₀-Bicycloalkylgruppe; eine C₃₋₁₀-Cycloalkenylgruppe; eine C₇₋₁₀-Bicycloalkenylgruppe; eine Arylgruppe; eine Heteroarylgruppe;
- R⁵ und R⁶, die gleich oder verschieden sind, Wasserstoff; [X]p-R⁷; C₁₋₆-Alkylen-SO₃Y; C₁₋₆-Alkylen-PO₃Y; C₁₋₆-Alkylen-N(R⁸)₃⁺ A-; eine geradkettige oder verzweigte C₁₋₂₀-Alkylgruppe; eine C₂₋₁₀-Alkenylgruppe; eine C₃-₁₀-Cycloalkylgruppe; eine C₇-₁₀-Bicycloalkylgruppe; eine C₃₋₁₀-Cydoalkenylgruppe; eine C₇-₁₀-Bicycloalkenylgruppe; eine Arylgruppe; eine Heteroarylgruppe;
- X eine Gruppe -CH₂-CH₂-Z-, -CH₂CH₂CH₂Z-, -CH(CH₃)-CH₂-Z-, -CH₂-CH₂-CH₂-CH₂-Z-, -CH₂-CH(CH₂CH₃)-Z-;
- A Cl, Br, I, SO₄R⁹;
- Y Wasserstoff, Na⁺, K⁺, Mg²⁺, Ca²⁺, Li⁺, Al³+, -N(R⁸)₄⁺;
- Z O oder NH;
- R⁷ und R⁸, die gleich oder verschieden sind, Wasserstoff; eine geradkettige oder verzweigte C₁-₆-Alkylgruppe; eine geradkettige oder verzweigte C₂-₆-Alkenylgruppe; eine geradkettige oder verzweigte C₁₋₆-Acylgruppe;
- R⁹ Wasserstoff; eine geradkettige oder verzweigte C₁-₆-Alkylgruppe; eine C₂-₆-Alkenylgruppe;
- n einen Wert von 1 bis 3;
- p einen Wert von 0 bis 150.

3. Zusammensetzung nach Anspruch 2, in der die Verbindung der Formel (I) unter Verbindungen der nachstehenden Formel (la) ausgewählt ist: 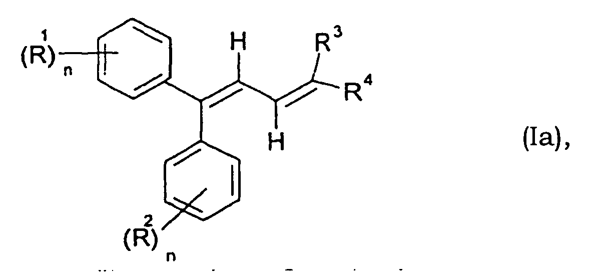 in der das Diensystem die Konfiguration ZZ; ZE; E,Z oder E,E aufweist oder Gemische dieser Konfigurationen vorliegen und wobei bedeuten:
- R¹ und R², die gleich oder verschieden sind, Wasserstoff; eine C₁₋₈-Alkylgruppe, eine C₁₋₈-Alkoxygruppe; einen wasserlöslich machenden Substituenten, der unter einer Carboxylatgruppe, einer Sulfonatgruppe und einem Ammoniumrest ausgewählt ist;
- R³ eine Gruppe COOR⁵; CONR⁵R⁶; CN;
- R⁴ eine Gruppe COOR⁶; CONR⁵R⁶;
- R⁵ Wasserstoff; [X]p-R⁷; C₁₋₆-Alkylen-SO₃Y; C₁₋₆-Alkylen-N(R⁸)₃⁺ A-;
- R⁶ [X]p-R⁷; C₁₋₆-Alkylen-SO₃Y; C₁₋₆-Alkylen-N(R⁸)₃⁺ A-;
- X eine Gruppe -CH₂-CH₂-O-, -CH₂CH₂CH₂O-, -CH(CH₃)-CH₂-O-;
- A Cl, Br, I, SO₄R⁹;
- Y Wasserstoff, Na⁺, K⁺, Mg²⁺, Ca²⁺, Li⁺, Al³⁺, -N(R⁸)₄+;
- R⁷, R⁸ und R⁹, die gleich oder verschieden sind, Wasserstoff; eine geradkettige oder verzweigte C₁₋₃-Alkylgruppe;
- n einen Wert von 1 bis 3;
- p einen Wert von 0 bis 50.

4. Zusammensetzung nach Anspruch 3, in der die Verbindung der Formel (I) unter Verbindungen der nachstehenden Formel (Ib) ausgewählt ist: 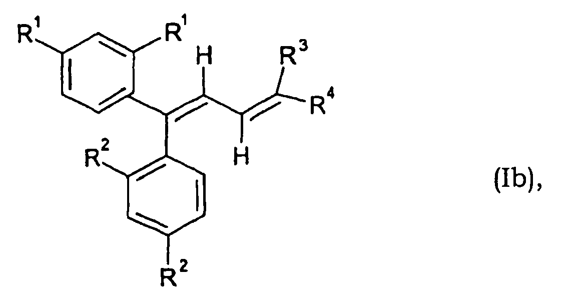 in der das Diensystem die Konfiguration ZZ; Z,E; E,Z oder E,E aufweist oder Gemische dieser Konfigurationen vorliegen und wobei bedeuten:
- R¹ und R², die gleich oder verschieden sind, Wasserstoff; eine C₁₋₈-Alkylgruppe; eine C₁₋₈-Alkoxygruppe;
- R³ eine Gruppe COOR⁵; CONR⁵R⁶; CN;
- R⁴ eine Gruppe COOR⁶; CONR⁵R⁶;
- R⁵ Wasserstoff; [X]ₚ-R⁷; C₁-₆-Alkylen-SO₃Y; C₁₋₆-Alkylen-N(R⁸)₃⁺ A-;
- R⁶ [X]ₚ-R⁷; C₁₋₆-Alkylen-SO₃Y; C₁₋₆-Alkylen-N(R⁸)₃⁺ A-;
- X eine Gruppe -CH₂-CH₂-O-, -CH₂CH₂CH₂O-, -CH(CH₃)-CH₂-O-;
- A Cl, Br, I, SO₄R⁹;
- Y Wasserstoff, Na⁺, K⁺, Mg²⁺, Ca²⁺, Li⁺, Al³⁺, -N(R⁸)₄+;
- R⁷, R⁸ und R⁹, die gleich oder verschieden sind, Wasserstoff; eine geradkettige oder verzweigte C₁₋₃-Alkylgruppe;
- p einen Wert von 0 bis 50.

5. Zusammensetzung nach Anspruch 4, in der die Verbindung der Formel (I) unter Verbindungen der nachstehenden Formel (Ic) ausgewählt ist: 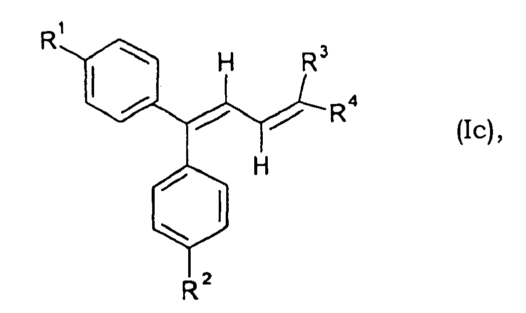 in der das Diensystem die Konfiguration Z,Z; Z,E; E,Z oder E,E aufweist oder Gemische dieser Konfigurationen vorliegen und wobei bedeuten:
- R¹ und R², die gleich oder verschieden sind, Wasserstoff; eine C₁₋₈-Alkylgruppe; eine C₁₋₈-Alkoxygruppe;
- R³ eine Gruppe COOR⁵; CONR⁵R⁶; CN;
- R⁴ eine Gruppe COOR⁶; CONR⁵R⁶;
- R⁵ Wasserstoff; [X]p-R⁷; C₁₋₆-Alkylen-SO₃Y; C₁₋₆-Alkylen-N(R⁸)₃⁺ A-;
- R⁶ [X]p-R⁷; C₁₋₆-Alkylen-SO₃Y; C₁₋₆-Alkylen-N(R⁸)₃+ A-;
- X eine Gruppe -CH₂-CH₂-O-, -CH₂CH₂CH₂O-, -CH(CH₃)-CH₂-O-;
- A Cl, Br, I, SO₄R⁹;
- Y Wasserstoff, Na⁺, K⁺, Mg²⁺, Ca²⁺, Li⁺, Al³⁺, -N(R⁸)₄+;
- R⁷, R⁸ und R⁹, die gleich oder verschieden sind, Wasserstoff; eine geradkettige oder verzweigte C₁₋₃-Alkylgruppe;
- p einen Wert von 0 bis 50.

6. Zusammensetzung nach Anspruch 4 oder 5, in der die Verbindung der Formel (I) unter folgenden Verbindungen ausgewählt ist:

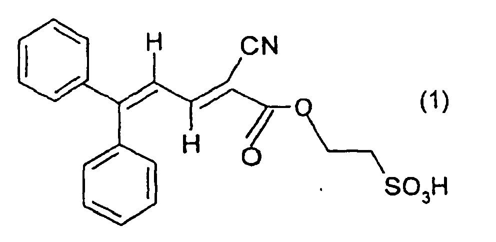

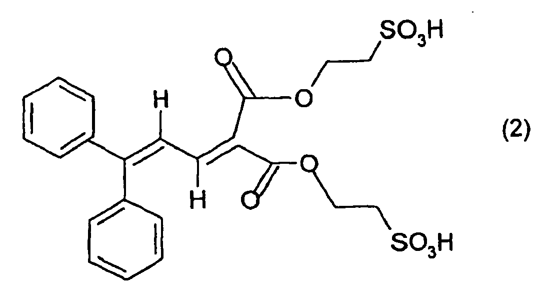

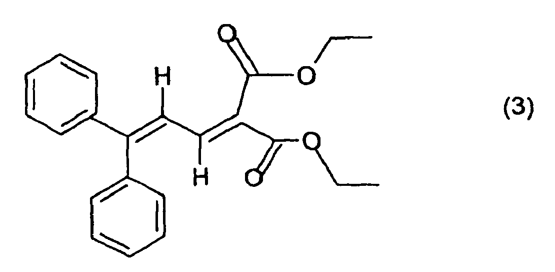

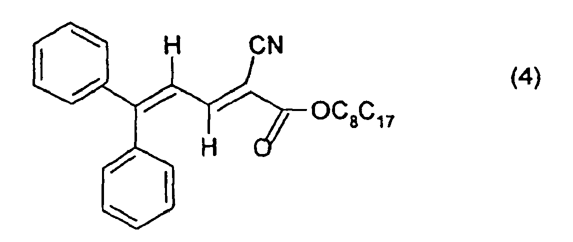

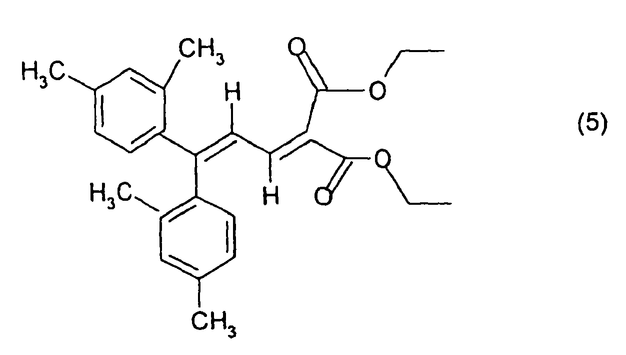

7. Zusammensetzung nach Anspruch 1, in der die 4,4-Diarylbutadien-Verbindung ein Oligomer der nachstehenden Formel (II) ist: 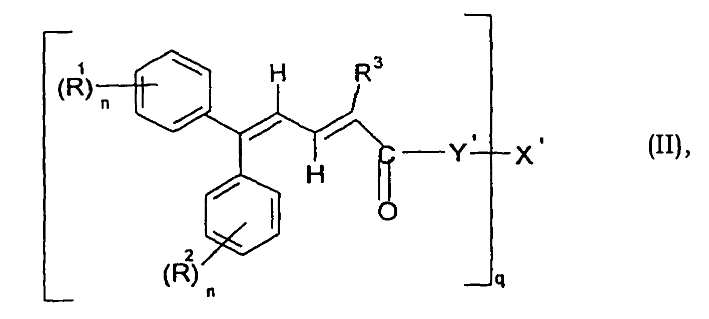 in der das Diensystem die Konfiguration Z,Z; Z,E; E,Z oder E,E aufweist oder Gemische dieser Konfigurationen vorliegen und wobei bedeuten:
- R¹, R², R³ und n dasselbe wie in Anspruch 2 für Formel (I) angegeben;
- Y' eine Gruppe -O- oder -NR¹⁰-;
- R¹⁰ Wasserstoff; eine geradkettige oder verzweigte C₁₋₂₀-Alkylgruppe; eine C₂₋₁₀-Alkenylgruppe; eine C₃₋₁₀-Cycloalkylgruppe; eine C₇₋₁₀-Bicycloalkylgruppe ; eine C₃₋₁₀-Cycloalkenylgruppe; eine C₇₋₁₀-Bicycloalkenylgruppe ; eine Arylgruppe; eine Heteroarylgruppe;
- X' eine geradkettige oder verzweigte, aliphatische oder cycloaliphatische Polyolgruppe, die 2 bis 10 Hydroxygruppen aufweist und die Wertigkeit q besitzt, wobei die Kohlenstoffkette dieser Gruppe durch ein oder mehrere Schwefelatome oder Sauerstoffatome unterbrochen sein kann; eine oder mehrere Iminogruppen oder eine oder mehrere C₁₋₄-Alkyliminogruppen;
- q einen Wert von 2 bis 10.

8. Zusammensetzung nach Anspruch 7, in der die Verbindung der Formel (II) ausgewählt ist unter Verbindungen, bei denen bedeuten:
- R¹ und R², die gleich oder verschieden sind, Wasserstoff; eine C₁₋₁₂-Alkylgruppe; eine C₁₋₈-Alkoxygruppe, einen wasserlöslich machenden Substituenten, der unter einer Carboxylatgruppe, einer Sulfonatgruppe oder einer Ammoniumgruppe ausgewählt ist;
- R³ eine Gruppe COOR⁵; CONR⁵R⁶; CN; eine C₃₋₁₀-Cycloalkylgruppe; eine C₇₋₁₀-Bicycloalkylgruppe;
- R⁵ und R⁶, die gleich oder verschieden sind, eine geradkettige oder verzweigte C₁₋₂₀-Alkylgruppe; eine C₃₋₁₀-Cycloalkylgruppe, eine C₇₋₁₀-Bicycloalkylgruppe, Naphthyl oder ggf. substituiertes Phenyl;
- X' eine Polyolgruppe, die 2 bis 6 Hydroxygruppen und insbesondere 2 bis 4 Hydroxygruppen aufweist.

9. Zusammensetzung nach Anspruch 8, in der die Verbindung der Formel (II) unter Verbindungen ausgewählt ist, bei denen
- X' einen Ethanolrest oder einen Pentaerythritrest bedeutet.

10. Zusammensetzung nach Anspruch 9, in der die Verbindung der Formel (II) unter den folgenden Verbindungen ausgewählt ist:

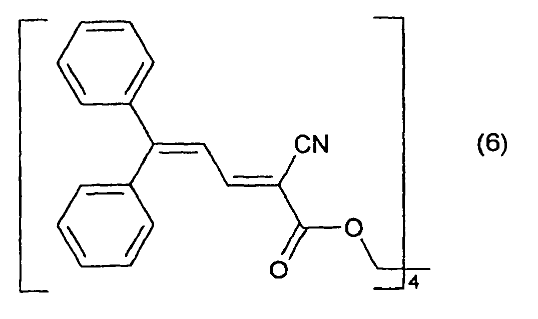

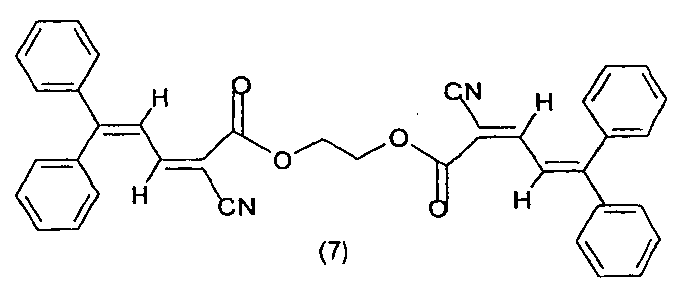

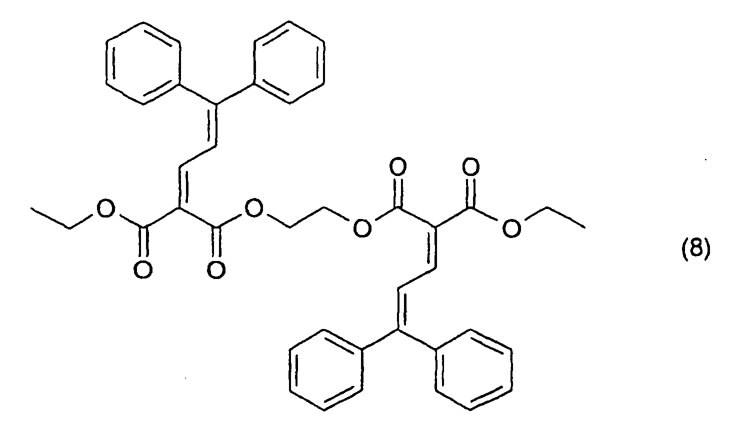

11. Zusammensetzung nach einem der Ansprüche 1 bis 10, **dadurch gekennzeichnet, dass** das Dibenzoylmethan-Derivat ausgewählt ist unter:
- 2-Methyldibenzoylmethan
- 4-Methyldibenzoylmethan
- 4-Isopropyldibenzoylmethan
- 4-tert.-Butyldibenzoylmethan
- 2,4-Dimethyldibenzoylmethan
- 2,5-Dimethyldibenzoylmethan
- 4,4'-Diisopropyldibenzoylmethan
- 4,4'-Dimethoxydibenzoylmethan
- 4-tert.-Butyl-4'-methoxydibenzoylmethan
- 2-Methyl-5-isopropyl-4'-methoxydibenzoylmethan
- 2-Methyl-5-tert.-butyl-4'-methoxydibenzoylmethan
- 2,4-Dimethyl-4'-methoxydibenzoylmethan
- 2,6-Dimethyl-4-tert.-butyl-4'-methoxydibenzoylmethan.

12. Zusammensetzung nach Anspruch 11, **dadurch gekennzeichnet, dass** das Dibenzoylmethan-Derivat 4-tert.-Butyl-4'-methoxy-dibenzoylmethan ist.

13. Zusammensetzung nach Anspruch 12, **dadurch gekennzeichnet, dass** das Dibenzoylmethan-Derivat 4-Isopropyldibenzoylmethan ist.

14. Zusammensetzung nach einem der Ansprüche 1 bis 13, in der die 4,4-Diarylbutadien-Verbindung in Mengenanteilen von 0,5 bis 15 Gew.-%, bezogen auf das Gesamtgewicht der Zusammensetzung, vorliegt.

15. Zusammensetzung nach Anspruch 14, in der die 4,4-Diarylbutadien-Verbindung in Mengenanteilen von 1 bis 10 Gew.-%, bezogen auf das Gesamtgewicht der Zusammensetzung, vorliegt.

16. Zusammensetzung nach einem der Ansprüche 1 bis 14, in der das Dibenzoylmethan-Derivat in Mengenanteilen von 0,5 bis 15 Gew.-%, bezogen auf das Gesamtgewicht der Zusammensetzung, vorliegt.

17. Zusammensetzung nach Anspruch 16, in der das Dibenzoylmethan-Derivat in Mengenanteilen von 1 bis 10 Gew.-%, bezogen auf das Gesamtgewicht der Zusammensetzung, vorliegt.

18. Zusammensetzung nach einem der Ansprüche 1 bis 17, **dadurch gekennzeichnet, dass** sie ferner weitere organische Filter enthält, die im UV-A-Bereich und/oder im UV-B-Bereich aktiv sind.

19. Zusammensetzung nach Anspruch 16, bei der das oder die zusätzlichen organischen UV-Filter ausgewählt sind unter den Anthranilaten; den Salicylsäurederivaten; den Campherderivaten; den Benzophenon-Derivaten; den β, β-Diphenylacrylat-Derivaten; den Benzotriazol-Derivaten; den Benzalmalonat-Derivaten; den Benzimidazol-Derivaten; den Imidazolinen; den Bis-benzoazolyl-Derivaten; den Derivaten der p-Aminobenzoesäure (PABA); den Derivaten von Methylen-bis(hydroxyphenylbenzotriazol); polymeren Filtern und Silicon-Filtern; den von α-Alkylstyrolen abgeleiteten Dimeren.

20. Zusammensetzung nach Anspruch 19, **dadurch gekennzeichnet, dass** das organische UV-Filter oder die organischen UV-Filter unter folgenden Verbindungen ausgewählt sind:
- Ethylhexyl Salicylate,
- Octocrylene,
- Phenylbenzimidazole Sulfonic Acid,
- Terephthalylidene Dicamphor Sulfonic Acid,
- Benzophenone-3,
- Benzophenone-4,
- Benzophenone-5,
- 4-Methylbenzylidene Camphor,
- Disodium Phenyl Dibenzimidazole Tetra-sulfonate,
- Anisotriazine,
- Ethylhexyl Triazone,
- Diethylhexyl Butamido Triazone,
- 2,4,6-Tris(4'-amino-benzaldüsobutylmalonat)-s-triazin,
- Methylene Bis-Benzotriazolyl Tetramethylbutylphenol,
- Drometrizole Trisiloxane
sowie Gemischen dieser Verbindungen.

21. Zusammensetzung nach einem der Ansprüche 1 bis 20, **dadurch gekennzeichnet, dass** sie ferner Pigmente oder Nanopigmente von Metalloxiden enthält, die gegebenenfalls ummantelt sind.

22. Zusammensetzung nach Anspruch 19, **dadurch gekennzeichnet, dass** die Pigmente oder Nanopigmente ausgewählt sind unter Titanoxid, Zinkoxid, Eisenoxid, Zirconiumoxid und Ceroxid sowie Gemischen dieser Verbindungen, wobei sie gegebenenfalls ummantelt sind.

23. Zusammensetzung nach einem der Ansprüche 1 bis 22, **dadurch gekennzeichnet, dass** sie ferner mindestens ein Mittel zur künstlichen Bräunung und/ oder künstlichen Braunfärbung der Haut enthält.

24. Zusammensetzung nach einem der Ansprüche 1 bis 23, **dadurch gekennzeichnet, dass** sie ferner mindestens einen Hilfsstoff enthält, der ausgewählt ist unter Fettsubstanzen, organischen Lösungsmitteln, ionischen oder nichtionischen Verdickungsmitteln, reizlindernden Mitteln, Antioxidantien, Mitteln gegen freie Radikale, Trübungsmitteln, Stabilisatoren, Emollientien, Siliconen, α-Hydroxysäuren, Antischaummitteln, hydratisierenden Mitteln, Vitaminen, Insektenrepellents, Parfums, Konservierungsmitteln, grenzflächenaktiven Mitteln, entzündungshemmenden Mitteln, Antagonisten der Substanz P, Füllstoffen, Polymeren, Treibmitteln, Mitteln zum Alkalischmachen oder zum Ansäuern, Färbemitteln.

25. Zusammensetzung nach einem der Ansprüche 1 bis 24, **dadurch gekennzeichnet, dass** es sich um eine Zusammensetzung zum Schutz der menschlichen Epidermis oder um eine Sonnenschutzzusammensetzung handelt und die Zusammensetzung in Form einer nichtionischen Vesikeldispersion, einer Emulsion, insbesondere einer Emulsion vom Öl-in-Wasser-Typ, einer Creme, einer Milch, eines Gels, einer Gelcreme, einer Suspension, einer Dispersion, eines Puders, eines festen Stiftes, eines Schaums oder eines Sprays vorliegt.

26. Zusammensetzung nach einem der Ansprüche 1 bis 24, **dadurch gekennzeichnet, dass** es sich um eine Zusammensetzung zum Schminken der Augenbrauen, der Wimpern oder der Haut handelt und die Zusammensetzung in fester oder pastoser Form, wasserfrei oder wasserhaltig, einer Emulsion, einer Suspension oder einer Dispersion vorliegt.

27. Zusammensetzung nach einem der Ansprüche 1 bis 24, **dadurch gekennzeichnet, dass** es sich um eine Zusammensetzung zum Schutz der Haare gegen ultraviolette Strahlung handelt und die Zusammensetzung in Form eines Shampoos, einer Lotion, eines Gels, einer Emulsion oder einer nichtionischen Vesikeldispersion vorliegt.

28. Verwendung einer 4,4-Diarylbutadien-Verbindung wie in einem der vorhergehenden Ansprüche definiert in einer kosmetischen Zusammensetzung, die mindestens ein UV-Filter vom Typ eines Dibenzoylmethan-Derivats enthält, um die Stabilität des Dibenzoylmethan-Derivats gegen UV-Strahlung zu verbessern, wobei das Gewichtsverhältnis von 4,4-Diarylbutadien-Verbindung zu Dibenzoylmethan-Derivat größer als 2,5 ist und die Zusammensetzung kein Cinnamat-Derivat enthält.

## Patentansprüche (Patentansprüche für folgende(n) Vertragsstaat(en): DE)

1. Kosmetische oder dermatologische Zusammensetzung zur topischen Anwendung zum Schutz der menschlichen Epidermis gegen UV-Strahlung, **dadurch gekennzeichnet, dass** sie in einem kosmetisch akzeptablen Träger enthält:
(a) mindestens ein UV-Filter vom Typ eines Dibenzoylmethan-Derivats und
(b) mindestens eine 4,4-Diarylbutadien-Verbindung,
wobei das Gewichtsverhältnis von 4,4-Diarylbutadien-Verbindung zu Dibenzoylmethan-Derivat größer als 2,5 ist und die Zusammensetzung kein Cinnamat-Derivat enthält,
und dass sie in Form einer nichtionischen Versikeldispersion, in Form einer Öl/Wasser-Emulsion, in Form eines Gels, in Form eines Pulders, in Form eines festen Stifts, in Form eines Aerosolschaums oder als Spray vorliegt.

2. Kosmetische oder dermatologische Zusammensetzung zur topischen Anwendung zum Schutz der Haare gegen UV-Strahlung, **dadurch gekennzeichnet, dass** sie in einem kosmetisch akzeptablen Träger enthält:
(a) mindestens ein UV-Filter vom Typ eines Dibenzoylmethan-Derivats und
(b) mindestens eine 4,4-Diarylbutadien-Verbindung,
wobei das Gewichtsverhältnis von 4,4-Diarylbutadien-Verbindung zu Dibenzoylmethan-Derivat größer als 2,5 ist und die Zusammensetzung kein Cinnamat-Derivat enthält,
und dass sie in Form eines Shampoos, eines Gels oder einer nichtionischen Versikeldispersion vorliegt.

3. Kosmetische oder dermatologische Zusammensetzung zum Schminken der Augenbrauen, der Wimpern oder der Haut, **dadurch gekennzeichnet, dass** sie in einem kosmetisch akzeptablen Träger enthält:
(a) mindestens ein UV-Filter vom Typ eines Dibenzoylmethan-Derivats und
(b) mindestens eine 4,4-Diarylbutadien-Verbindung,
wobei das Gewichtsverhältnis von 4,4-Diarylbutadien-Verbindung zu Dibenzoylmethan-Derivat größer als 2,5 ist und die Zusammensetzung kein Cinnamat-Derivat enthält,
und dass sie in fester oder pastoser Form, wasserfrei oder in wässeriger Form, in Form einer Öl/Wasser-Emulsion oder in Form einer nichtionischen Versikeldispersion vorliegt.

4. Zusammensetzung nach einem der Ansprüche 1 bis 3, in der die 4,4-Diarylbutadien-Verbindung die nachstehende Formel (I) aufweist: 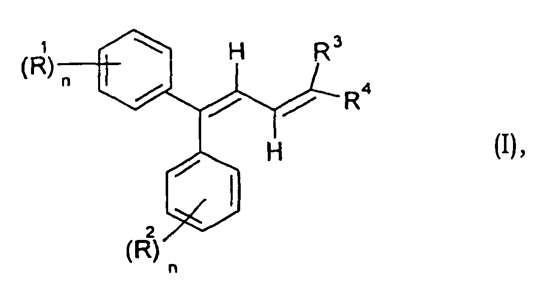 in der das Diensystem die Konfiguration Z,Z; Z,E; E,Z oder E,E aufweist oder Gemische dieser Konfigurationen vorliegen und wobei bedeuten:
- R¹ und R², die gleich oder verschieden sind, Wasserstoff; eine geradkettige oder verzweigte C₁₋₂₀-Alkylgruppe; eine C₂₋₁₀-Alkenylgruppe; eine C₁₋₁₂-Alkoxvgruppe; eine C₃₋₁₀-Cycloalkylgruppe; eine C₃₋₁₀-Cycloalkenylgruppe; eine geradkettige oder verzweigte C₁₋₂₀-Alkoxycarbonylgruppe; eine geradkettige oder verzweigte C₁₋₁₂-Monoalkylaminogruppe; eine geradkettige oder verzweigte C₁₋₁₂-Dialkylaminogruppe; eine Arylgruppe, eine Heteroarylgruppe oder einen wasserlöslich machenden Substituenten, der unter einer Carboxylatgruppe, einer Sulfonatgruppe und einem Ammoniumrest ausgewählt ist;
- R³ eine Gruppe COOR⁵; COR⁵; CONR⁵R⁶; CN; eine geradkettige oder verzweigte C₁₋₂₀-Alkylgruppe; eine C₂₋₁₀-Alkenylgruppe, eine C₃₋₁₀-Cycloalkylgruppe; eine C₇₋₁₀-Bicycloalkylgruppe, eine C₃₋₁₀-Cycloalkenylgruppe, eine C₇₋₁₀-Bicycloalkenylgruppe; eine C₆₋₁₈-Arylgruppe; eine C₃₋₇-Heteroarylgruppe;
- R⁴ eine Gruppe COOR⁶; COR⁶; CONR⁵R⁶; CN; eine geradkettige oder verzweigte C₁₋₂₀-Alkylgruppe, eine C₂-₁₀-Alkenylgruppe; eine C₃₋₁₀-Cycloalkylgruppe; eine C₇₋₁₀-Bicycloalkylgruppe; eine C₃₋₁₀-Cycloalkenylgruppe; eine C₇₋₁₀-Bicycloalkenylgruppe; eine Arylgruppe; eine Heteroarylgruppe;
- R⁵ und R⁶, die gleich oder verschieden sind, Wasserstoff; [X]ₚ-R⁷; C₁₋₆-Alkylen-SO₃Y; C₁₋₆-Alkylen-PO₃Y; C₁₋₆-Alkylen-N(R⁸)₃⁺ A-; eine geradkettige oder verzweigte C₁₋₂₀-Alkylgruppe; eine C₂₋₁₀-Alkenylgruppe; eine C₃₋₁₀-Cycloalkylgruppe; eine C₇₋₁₀-Bicycloalkylgruppe; eine C₃₋₁₀-Cycloalkenylgruppe; eine C₇₋₁₀-Bicycloalkenylgruppe; eine Arylgruppe; eine Heteroarylgruppe;
- X eine Gruppe -CH₂-CH₂-Z-, -CH₂CH₂CH₂Z-, -CH(CH₃)-CH₂-Z-, -CH₂-CH₂-CH₂-CH₂-Z-, -CH₂-CH(CH₂CH₃)-Z-;
- A Cl, Br, I, SO₄R⁹;
- Y Wasserstoff, Na⁺, K⁺, Mg²⁺, Ca²⁺, Li⁺, Al³⁺ -N(R8)₄⁺;
- Z O oder NH;
- R⁷ und R⁸, die gleich oder verschieden sind, Wasserstoff; eine geradkettige oder verzweigte C₁₋₆-Alkylgruppe; eine geradkettige oder verzweigte C₂₋₆-Alkenylgruppe; eine geradkettige oder verzweigte C₁₋₆-Acylgruppe;
- R⁹ Wasserstoff; eine geradkettige oder verzweigte C₁₋₆-Alkylgruppe; eine C₂₋₆-Alkenylgruppe;
- n einen Wert von 1 bis 3;
- p einen Wert von 0 bis 150.

5. Zusammensetzung nach Anspruch 4, in der die Verbindung der Formel (I) unter Verbindungen der nachstehenden Formel (Ia) ausgewählt ist: 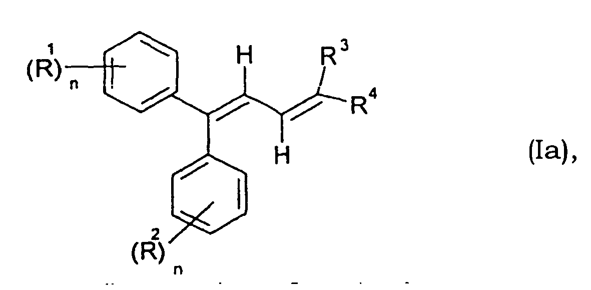 in der das Diensystem die Konfiguration ZZ; ZE; E,Z oder E,E aufweist oder Gemische dieser Konfigurationen vorliegen und wobei bedeuten:
- R¹ und R², die gleich oder verschieden sind, Wasserstoff; eine C₁₋₈-Alkylgruppe, eine C₁₋₈-Alkoxygruppe; einen wasserlöslich machenden Substituenten, der unter einer Carboxylatgruppe, einer Sulfonatgruppe und einem Ammoniumrest ausgewählt ist;
- R³ eine Gruppe COOR⁵; CONR⁵R⁶; CN;
- R⁴ eine Gruppe COOR⁶; CONR⁵R⁶;
- R⁵ Wasserstoff; [X]p-R⁷; C₁₋₆-Alkylen-SO₃Y; C₁₋₆-Alkylen-N(R⁸)₃⁺ A-;
- R⁶ [X]p-R⁷; C₁₋₆-Alkylen-SO₃Y; C₁₋₆-Alkylen-N(R⁸)₃⁺ A-;
- X eine Gruppe -CH₂-CH₂-O-, -CH₂CH₂CH₂O-, -CH(CH₃)-CH₂-O-;
- A Cl, Br, I, SO₄R⁹;
- Y Wasserstoff, Na⁺, K⁺, Mg²⁺, Ca²⁺, Li⁺, Al³⁺, -N(R8)₄+;
- R⁷, R⁸ und R⁹, die gleich oder verschieden sind, Wasserstoff; eine geradkettige oder verzweigte C₁₋₃-Alkylgruppe;
- n einen Wert von 1 bis 3;
- p einen Wert von 0 bis 50.

6. Zusammensetzung nach Anspruch 5, in der die Verbindung der Formel (I) unter Verbindungen der nachstehenden Formel (Ib) ausgewählt ist: 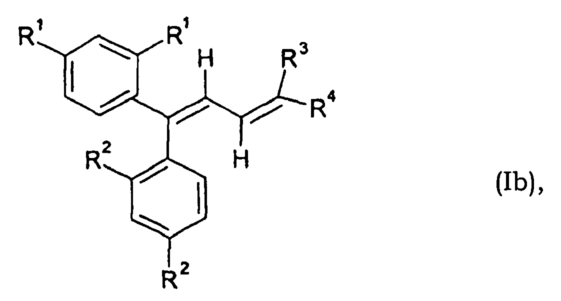 in der das Diensystem die Konfiguration ZZ; Z,E; E,Z oder E,E aufweist oder Gemische dieser Konfigurationen vorliegen und wobei bedeuten:
- R¹ und R², die gleich oder verschieden sind, Wasserstoff; eine C₁₋₈-Alkylgruppe; eine C₁₋₈-Alkoxygruppe;
- R³ eine Gruppe COOR⁵; CONR⁵R⁶; CN;
- R⁴ eine Gruppe COOR⁶; CONR⁵R⁶;
- R⁵ Wasserstoff; [X]p-R⁷; C₁₋₆-Alkylen-SO₃Y; C₁₋₆-Alkylen-N(R⁸)₃⁺ A-;
- R⁶ [X]p-R⁷; C₁₋₆-Alkylen-SO₃Y; C₁₋₆-Alkylen-N(R⁸)₃⁺ A-;
- X eine Gruppe -CH₂-CH₂-O-, -CH₂CH₂CH₂O-, -CH(CH₃)-CH₂-O-;
- A Cl, Br, I, SO₄R⁹;
- Y Wasserstoff, Na⁺, K⁺, Mg²⁺, Ca²⁺, Li⁺, Al³⁺, -N(R8)₄⁺;
- R⁷, R⁸ und R⁹, die gleich oder verschieden sind, Wasserstoff; eine geradkettige oder verzweigte C₁₋₃-Alkylgruppe;
- p einen Wert von 0 bis 50.

7. Zusammensetzung nach Anspruch 6, in der die Verbindung der Formel (I) unter Verbindungen der nachstehenden Formel (Ic) ausgewählt ist: 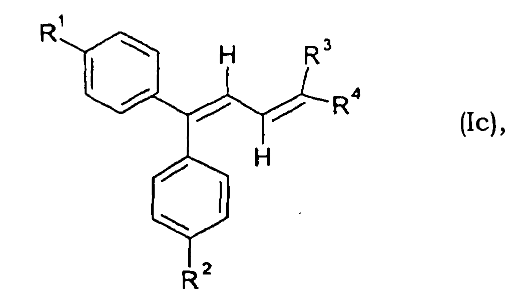 in der das Diensystem die Konfiguration Z,Z; Z,E; E,Z oder E,E aufweist oder Gemische dieser Konfigurationen vorliegen und wobei bedeuten:
- R¹ und R², die gleich oder verschieden sind, Wasserstoff; eine C₁-₈-Alkylgruppe; eine C₁₋₈-Alkoxygruppe;
- R³ eine Gruppe COOR⁵; CONR⁵R⁶; CN;
- R⁴ eine Gruppe COOR⁶; CONR⁵R⁶;
- R⁵ Wasserstoff; [X]p-R⁷; C₁₋₆-Alkylen-SO₃Y; C₁₋₆-Alkylen-N(R⁸)₃⁺ A-;
- R⁶ [X]p-R⁷; C₁₋₆-Alkylen-SO₃Y; C₁₋₆-Alkylen-N(R⁸)₃⁺ A-;
- X eine Gruppe -CH₂-CH₂-O-, -CH₂CH₂CH₂O-, -CH(CH₃)-CH₂-O-;
- A Cl, Br, I, SO₄R⁹;
- Y Wasserstoff, Na⁺, K⁺, Mg²⁺, Ca²⁺, Li⁺, Al³⁺, -N(R⁸)₄⁺ ;
- R⁷, R⁸ und R⁹, die gleich oder verschieden sind, Wasserstoff; eine geradkettige oder verzweigte C₁₋₃-Alkylgruppe;
- p einen Wert von 0 bis 50.

8. Zusammensetzung nach Anspruch 6 oder 7, in der die Verbindung der Formel (I) unter folgenden Verbindungen ausgewählt ist:

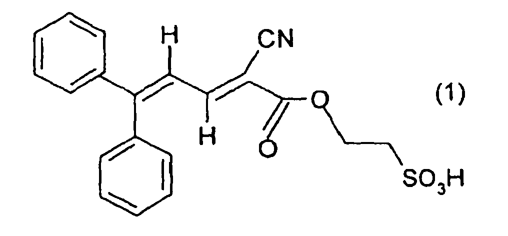

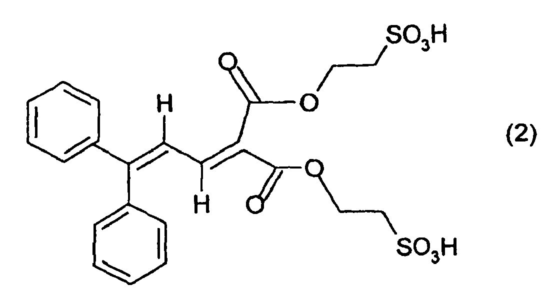

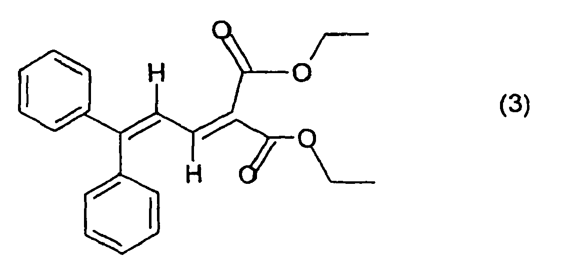

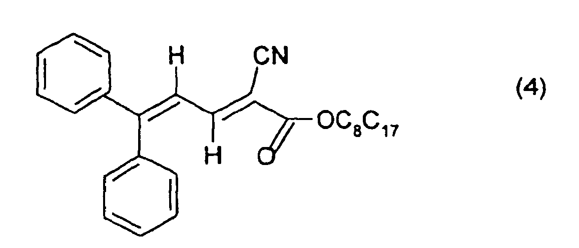

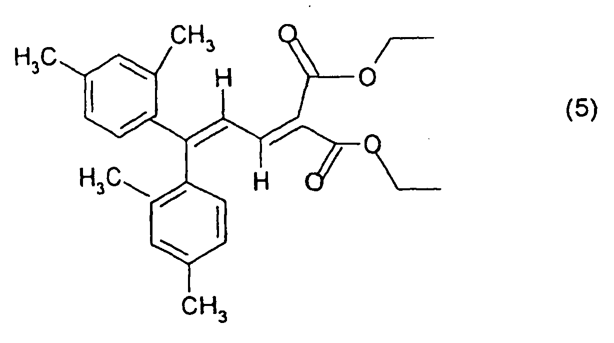

9. Zusammensetzung nach einem der Ansprüche 1 bis 3, in der die 4,4-Diarylbutadien-Verbindung ein Oligomer der nachstehenden Formel (II) ist: 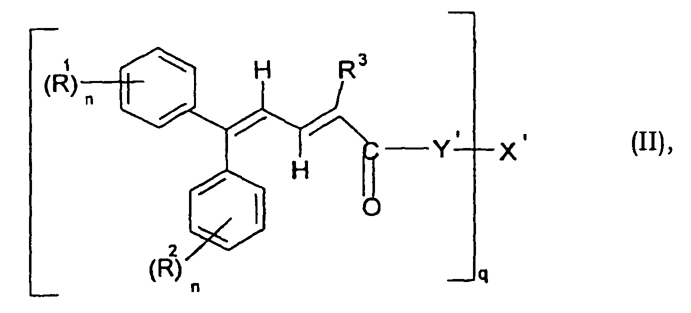 in der das Diensystem die Konfiguration Z,Z; Z,E; E,Z oder E,E aufweist oder Gemische dieser Konfigurationen vorliegen und wobei bedeuten:
- R¹, R², R³ und n dasselbe wie in Anspruch 4 für Formel (I) angegeben;
- Y' eine Gruppe -O- oder -NR¹⁰-;
- R¹⁰ Wasserstoff; eine geradkettige oder verzweigte C₁₋₂₀-Alkylgruppe; eine C₂₋₁₀-Alkenylgruppe; eine C₃₋₁₀-Cycloalkylgruppe; eine C₇₋₁₀-Bicycloalkylgruppe; eine C₃₋₁₀-Cycloalkenylgruppe; eine C₇₋₁₀-Bicycloalkenylgruppe; eine Arylgruppe; eine Heteroarylgruppe;
- X' eine geradkettige oder verzweigte, aliphatische oder cycloaliphatische Polyolgruppe, die 2 bis 10 Hydroxygruppen aufweist und die Wertigkeit q besitzt, wobei die Kohlenstoffkette dieser Gruppe durch ein oder mehrere Schwefelatome oder Sauerstoffatome unterbrochen sein kann; eine oder mehrere Iminogruppen oder eine oder mehrere C₁₋₄-Alkyliminogruppen;
- q einen Wert von 2 bis 10.

10. Zusammensetzung nach Anspruch 9, in der die Verbindung der Formel (II) ausgewählt ist unter Verbindungen, bei denen bedeuten:
- R¹ und R², die gleich oder verschieden sind, Wasserstoff; eine C₁₋₁₂-Alkylgruppe; eine C₁₋₈-Alkoxygruppe, einen wasserlöslich machenden Substituenten, der unter einer Carboxylatgruppe, einer Sulfonatgruppe oder einer Ammoniumgruppe ausgewählt ist;
- R³ eine Gruppe COOR⁵; CONR⁵R⁶; CN; eine C₃₋₁₀-Cycloalkylgruppe; eine C₇₋₁₀-Bicycloalkylgruppe;
- R⁵ und R⁶, die gleich oder verschieden sind, eine geradkettige oder verzweigte C₁₋₂₀-Alkylgruppe; eine C₃₋₁₀-Cycloalkylgruppe, eine C₇₋₁₀-Bicycloalkylgruppe, Naphthyl oder ggf. substituiertes Phenyl;
- X' eine Polyolgruppe, die 2 bis 6 Hydroxygruppen und insbesondere 2 bis 4 Hydroxygruppen aufweist.

11. Zusammensetzung nach Anspruch 10, in der die Verbindung der Formel (II) unter Verbindungen ausgewählt ist, bei denen
- X' einen Ethanolrest oder einen Pentaerythritrest bedeutet.

12. Zusammensetzung nach Anspruch 11, in der die Verbindung der Formel (II) unter den folgenden Verbindungen ausgewählt ist:

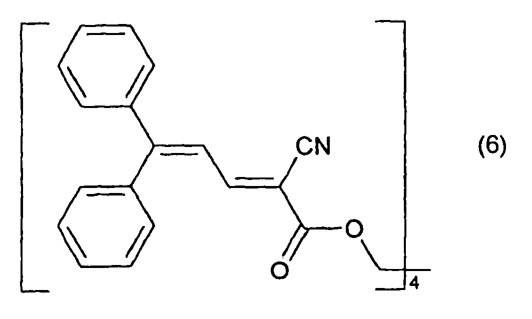

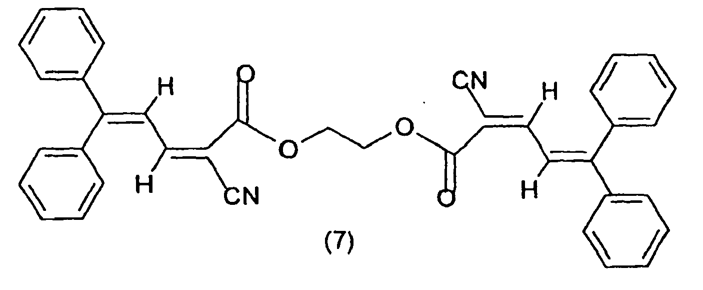

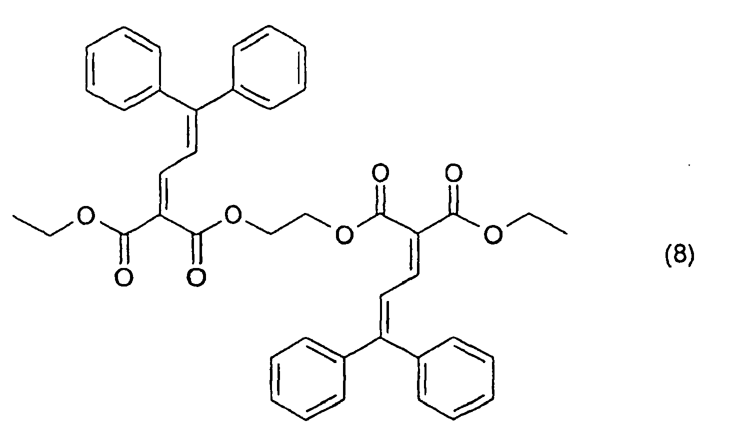

13. Zusammensetzung nach einem der Ansprüche 1 bis 12, **dadurch gekennzeichnet, dass** das Dibenzoylmethan-Derivat ausgewählt ist unter:
- 2-Methyldibenzoylmethan
- 4-Methyldibenzoylmethan
- 4-Isopropyldibenzoylmethan
- 4-tert.-Butyldibenzoylmethan
- 2,4-Dimethyldibenzoylmethan
- 2,5-Dimethyldibenzoylmethan
- 4,4'-Diisopropyldibenzoylmethan
- 4,4'-Dimethoxydibenzoylmethan
- 4-tert.-Butyl-4'-methoxydibenzoylmethan
- 2-Methyl-5-isopropyl-4'-methoxydibenzoylmethan
- 2-Methyl-5-tert.-butyl-4'-methoxydibenzoylmethan
- 2,4-Dimethyl-4'-methoxydibenzoylmethan
- 2,6-Dimethyl-4-tert.-butyl-4'-methoxydibenzoylmethan.

14. Zusammensetzung nach Anspruch 13, **dadurch gekennzeichnet, dass** das Dibenzoylmethan-Derivat 4-tert.-Butyl-4'-methoxy-dibenzoylmethan ist.

15. Zusammensetzung nach Anspruch 13, **dadurch gekennzeichnet, dass** das Dibenzoylmethan-Derivat 4-Isopropyldibenzoylmethan ist.

16. Zusammensetzung nach einem der Ansprüche 1 bis 15, in der die 4,4-Diarylbutadien-Verbindung in Mengenanteilen von 0,5 bis 15 Gew.-%, bezogen auf das Gesamtgewicht der Zusammensetzung, vorliegt.

17. Zusammensetzung nach Anspruch 16, in der die 4,4-Diarylbutadien-Verbindung in Mengenanteilen von 1 bis 10 Gew.-%, bezogen auf das Gesamtgewicht der Zusammensetzung, vorliegt.

18. Zusammensetzung nach einem der Ansprüche 1 bis 17, in der das Dibenzoylmethan-Derivat in Mengenanteilen von 0,5 bis 15 Gew.-%, bezogen auf das Gesamtgewicht der Zusammensetzung, vorliegt.

19. Zusammensetzung nach Anspruch 18, in der das Dibenzoylmethan-Derivat in Mengenanteilen von 1 bis 10 Gew.-%, bezogen auf das Gesamtgewicht der Zusammensetzung, vorliegt.

20. Zusammensetzung nach einem der Ansprüche 1 bis 19, **dadurch gekennzeichnet, dass** sie ferner weitere organische Filter enthält, die im UV-A-Bereich und/oder im UV-B-Bereich aktiv sind.

21. Zusammensetzung nach Anspruch 20, bei der das oder die zusätzlichen organischen UV-Filter ausgewählt sind unter den Anthranilaten; den Salicylsäurederivaten; den Campherderivaten; den Benzophenon-Derivaten; den β, β-Diphenylacrylat-Derivaten; den Benzotriazol-Derivaten; den Benzalmalonat-Derivaten; den Benzimidazol-Derivaten; den Imidazolinen; den Bis-benzoazolyl-Derivaten; den Derivaten der p-Aminobenzoesäure (PABA); den Derivaten von Methylen-bis(hydroxyphenyl-benzotriazol); polymeren Filtern und Silicon-Filtern; den von α-Alkylstyrolen abgeleiteten Dimeren.

22. Zusammensetzung nach Anspruch 21, **dadurch gekennzeichnet, dass** das organische UV-Filter oder die organischen UV-Filter unter folgenden Verbindungen ausgewählt sind:
- Ethylhexyl Salicylate,
- Octocrylene,
- Phenylbenzimidazole Sulfonic Acid,
- Terephthalylidene Dicamphor Sulfonic Acid,
- Benzophenone-3,
- Benzophenone-4,
- Benzophenone-5,
- 4-Methylbenzylidene Camphor,
- Disodium Phenyl Dibenzimidazole Tetra-sulfonate,
- Anisotriazine,
- Ethylhexyl Triazone,
- Diethylhexyl Butamido Triazone,
- 2,4,6-Tris(4'-amino-benzaldüsobutylmalonat)-s-triazin,
- Methylene Bis-Benzotriazolyl Tetramethylbutylphenol,
- Drometrizole Trisiloxane
sowie Gemischen dieser Verbindungen.

23. Zusammensetzung nach einem der Ansprüche 1 bis 22, **dadurch gekennzeichnet, dass** sie ferner Pigmente oder Nanopigmente von Metalloxiden enthält, die gegebenenfalls ummantelt sind.

24. Zusammensetzung nach Anspruch 23, **dadurch gekennzeichnet, dass** die Pigmente oder Nanopigmente ausgewählt sind unter Titanoxid, Zinkoxid, Eisenoxid, Zirconiumoxid und Ceroxid sowie Gemischen dieser Verbindungen, wobei sie gegebenenfalls ummantelt sind.

25. Zusammensetzung nach einem der Ansprüche 1 bis 24, **dadurch gekennzeichnet, dass** sie ferner mindestens ein Mittel zur künstlichen Bräunung und/oder künstlichen Braunfärbung der Haut enthält.

26. Zusammensetzung nach einem der Ansprüche 1 bis 25, **dadurch gekennzeichnet, dass** sie ferner mindestens einen Hilfsstoff enthält, der ausgewählt ist unter Fettsubstanzen, organischen Lösungsmitteln, ionischen oder nichtionischen Verdickungsmitteln, reizlindernden Mitteln, Antioxidantien, Mitteln gegen freie Radikale, Trübungsmitteln, Stabilisatoren, Emollientien, Siliconen, α-Hydroxysäuren, Antischaummitteln, hydratisierenden Mitteln, Vitaminen, Insektenrepellents, Parfums, Konservierungsmitteln, grenzflächenaktiven Mitteln, entzündungshemmenden Mitteln, Antagonisten der Substanz P, Füllstoffen, Polymeren, Treibmitteln, Mitteln zum Alkalischmachen oder zum Ansäuern, Färbemitteln.

27. Verwendung einer 4,4-Diarylbutadien-Verbindung wie in einem der vorhergehenden Ansprüche definiert in einer kosmetischen Zusammensetzung, die mindestens ein UV-Filter vom Typ eines Dibenzoylmethan-Derivats enthält, um die Stabilität des Dibenzoylmethan-Derivats gegen UV-Strahlung zu verbessern, wobei das Gewichtsverhältnis von 4,4-Diarylbutadien-Verbindung zu Dibenzoylmethan-Derivat größer als 2,5 ist und die Zusammensetzung kein Cinnamat-Derivat enthält.
